# EUROPÄISCHE PATENTSCHRIFT

(11) **EP 0 833 942 B1**
(45) Veröffentlichungstag und Bekanntmachung des Hinweises auf die Patenterteilung: **30.08.2000**
(21) Anmeldenummer: 96915904.5
(22) Anmeldetag: 13.06.1996
(51) Int. Cl.: C12Q 1/68

(54) **VERFAHREN ZUR TRANSKRIPTIONSFREIEN AMPLIFIZIERUNG VON NUKLEINSÄUREN**
PROCESS FOR THE TRANSCRIPTIONLESS AMPLIFICATION OF NUCLEIC ACIDS
PROCEDE D'AMPLIFICATION SANS TRANSCRIPTION D'ACIDES NUCLEIQUES

(30) Priorität: 13.06.1995 AT 100795
(43) Veröffentlichungstag der Anmeldung: 08.04.1998
(73) Patentinhaber: Himmler, Gottfried, 1180 Wien (AT); SCHLEDERER, Thomas, A-1220 Wien (AT)
(72) Erfinder: Himmler, Gottfried, 1180 Wien (AT); SCHLEDERER, Thomas, A-1220 Wien (AT)
(74) Vertreter: Pawloy, Peter Michael, Dipl.-Ing.
(86) Internationale Anmeldenummer: AT9600106
(87) Internationale Veröffentlichungsnummer: WO9700330

(56) Entgegenhaltungen:
- EP-A- 0 497 272
- WO-A-90/01069
- WO-A-94/03635
- WO-A-95/25180
- US-A- 5 273 881
- CRITICAL REVIEWS IN BIOCHEMISTRY AND MOLECULAR BIOLOGY, Bd. 26, Nr. 3/4, 1991, Seiten 227-259, XP000616490 WETMUR: "DNA probes: application of the principles of nucleic acid hybridization"
- J.BIOL.CHEM., Bd. 269, Nr. 13, 1.April 1994, Seiten 10163-10168, XP002024694 KMIEC AND HOLLOMAN: "DNA strand exchange in the absence of homologous pairing"
- BIOCONJUGATE CHEM., Bd. 6, Februar 1995, Seiten 93-100, XP002024695 COREY ET AL.: "Strand invasion by oligonucleotide-nuclease conjugates"
- JOURNAL OF BIOLOGICAL CHEMISTRY, Bd. 270, Nr. 24, 16.Juni 1995, Seiten 14712-14717, XP000601663 IYER M ET AL: "ACCELERATED HYBRIDIZATION OF OLIGONUCLEOTIDES TO DUPLEX DNA" in der Anmeldung erwähnt

## Beschreibung

**Gebiet der Erfindung**: Die vorliegende Erfindung bezieht sich auf das Gebiet der Molekularbiologie und der rekombinanten DNA-Technologie (Gentechnik). Die Erfindung bietet Möglichkeiten zur vereinfachten in-vitro- Synthese von Nukleinsäuren. Im Besonderen sind die hier beschriebenen Methoden auf die Nukleinsäureamplifikation mit Primer-abhängigen DNA/RNA Polymerasen, DNA- und RNA-Ligasen anwendbar. Die Erfindung hat eine Vielzahl von Anwendungen in der Molekularbiologie, der medizinischen Diagnose, der Umwelt- und der forensischen Analytik.

Insbesonders betrifft die vorliegende Erfindung ein Verfahren zur isothermen, transkriptionsfreien Amplifizierung von Nukleinsäuren bzw. Nukleinsäuresequenzen mittels Enzymen, wobei die Nukleinsäuren bzw. Nukleinsäuresequenzen gegebenenfalls vor der Amplifizierung zumindest teilweise in ihre Einzelstränge aufgetrennt und/oder transkribiert werden können.

Die Amplifizierung erfolgt durch enzymatischen Einbau von mindestens zwei Oligonukleotidbausteinen mit einer Basensequenz im wesentlichen komplementär zu den Basensequenzen der jeweiligen Enden der komplementären Stränge der zu amplifizierenden Nukleinsäure bzw. Nukleinsäuresequenz, wobei das Produkt aus den Bausteinen selbst wieder der zu amplifizierenden Nukleinsäure bzw. Nukleinsäuresequenz entspricht.

**Stand der Technik**: Die Amplifikation von Nukleinsäuren, wie z.B. DNA und RNA, bzw. Nukleinsäuresequenzen in vitro hat viele Anwendungen und kann auf verschiedene Art und Weise durchgeführt werden (wenn im folgenden von "Amplifizierung" die Rede ist, so ist darunter eine wesentliche Vermehrung, d.h. mehr als Verdopplung der Ausgangsprodukte zu verstehen).

Das Prinzip der Vermehrung von DNA mittels Polymeraseaktivität wurde von Kleppe et al., J. Mol. Biol., 56:341 (1971) im Detail beschrieben. Danach werden Oligonukleotide als Startpunkte für die enzymatische DNA-Synthese verwendet, sodaß das Produkt dieser Synthese wieder als Template für eine weitere Synthese dienen kann. Nach diesem Prinzip müssen zwischen den einzelnen Syntheseschritten die komplementären Nukleinsäurestränge immer wieder voneinander getrennt werden. Die US-PS 4683202 beschreibt die praktische Umsetzung dieses Prinzips.

Hinsichtlich der angewandten Temperatur sind dabei insbesondere zwei Verfahren zu unterscheiden, nämlich Verfahren mit zyklischer Temperaturänderung zwischen einer Amplifizierungs- und einer Denaturierungs (Strangauftrennungs-)temperatur, wobei die Nukleinsäuren bzw. Nukleinsäuresequenzen vor jedem Amplifizierungsschritt praktisch vollständig in ihre Einzelstränge zerlegt werden, sowie isotherme Verfahren.

Zur ersteren Gruppe der Temperatur-Cycling-Verfahren gehört beispielsweise das sogenannte PCR ("Polymerase Chain Reaction")-Verfahren, wobei eine Nukleinsäuresequenz in einer Reaktion, bei der die Temperatur einer zyklischen Änderung unterworfen wird, in exponentieller Form vermehrt werden kann. Für die Amplifizierung der Nukleinsäuresequenz wird meist eine thermostabile Polymerase verwendet [vgl. z.B. Saiki et al., Science, 230, 1350-1354 (1985) bzw. Saiki et al., Science, 239, 487, (1988)].

Gemäß der US-PS 4683195 (Mullis et al.) kann in einem Temperatur-Cycling Verfahren die an den Amplifizierungsschritt anschließende Strangtrennung durch jede passende Denaturierungsmethode erreicht werden, sei sie chemischer, physikalischer oder enzymatischer Natur. In dem genannten Dokument werden physikalische Strangtrennungsmethoden bevorzugt, z.B. Erhitzen der Nukleinsäure bis sie komplett (>99%) denaturiert, d.h. in die beiden Einzelstränge zerlegt ist. Eine typische Hitzedenaturierung erfolgt bei Temperaturen zwischen 90 °C und 105°C und dauert allgemein zwischen 0,5 und 5 Minuten. Bevorzugte Temperaturen sind zwischen 90 ° und 100°C für 0,5 bis 3 Minuten. Bei Verwendung eines thermolabilen Enzyms [US-PS 4683202] muß dabei nach jeder Strangtrennung durch Hitze frisches Enzym zugesetzt werden. Durch Verwendung eines thermostabilen Enzymes kann das Unterbrechen des Temperaturcyclings zum Zweck der Enzymzugabe unterbleiben, der Prozeß läuft mit Hilfe entsprechender Temperaturcycling-Automaten "simultan" ab.

Ein weiteres Verfahren aus der Gruppe der Temperatur-Cycling-Verfahren ist das sogenannte LCR ("Ligase Chain Reaction")- Verfahren, etwa gemäß F. Barany in Proc. Natl. Acad. Sci. USA, 88, 189-193, 1991, wobei diese Reaktion wie die PCR zu einer nichtlinearen Vermehrung der eingesetzten Nukleinsäure führt, auch eine zyklische Temperaturänderung zwischen einer Amplifizierungs- und, einer Denaturierungstemperatur erfolgt und thermostabile Ligase als Enzym verwendet wird. (WO 90/01069 und EP-A 320308). Ein spezieller Fall der LCR ist die sogenannte "gap-filling LCR", die zusätzlich zur Ligase auch eine Polymerase verwendet, bei der nur die Produkte aus der Polymerasereaktion als Substrate für die Ligase verwendet werden (US-PS 5427930).

Es können auch mehrere ähnliche Enzyme mit leicht unterschiedlichen Eigenschaften in einer Reaktion verwendet werden werden, um besondere Ergebnisse zu erzielen, die mit einem Enzym nicht erreicht werden können. Die Amplifizierung langer DNA-Abschnitte ist ein Beispiel für eine derartige Reaktion (Barnes, W.M. Proc. Natl. Acad. Sci. USA,91, 2216-2220, 1994).

Andererseits sind auch isotherme Verfahren, wie z.B. die "self sustained sequence replication" (3SR) im Stand der Technik bekannt, beispielsweise aus: E. Fahy et al., PCR Meth. Appl. 1, 25-33, 1991; D. Kwoh et al., Proc. Natl. Acad. Sci. (USA) 86, 1173-7 (1989); der WO 88/10315 (T. R. Gingeras et al.); der EP-A 0329822 (H.I. Miller et al.); und aus J. van Brunt, Bio/Technology 8, 291-4 (1990); US-P S 5409818, 5399491 und 5194370).

Diesen Verfahren ist gemeinsam, daß sie zwar isotherm durchgeführt werden können, für die Amplifizierung jedoch mehrere verschiedene Enzyme notwendig sind (z.B. Reverse Transkriptase, RNaseH, RNA-Polymerase), da der Amplifizierungsschritt über eine Transkription (RNA zu DNA bzw. DNA zu RNA) verläuft. Nachteilig ist auch, daß die isothermen Verfahren des Standes der Technik niedrigere Spezifität aufweisen, da keine thermostabilen Enzyme verwendet werden. Wenn RNA-Produkte das Ergebnis sind, besteht ein weiterer Nachteil darin, daß diese nur schwer handzuhaben sind (die Größenauftrennung ist nicht so einfach wie bei DNA). Weiters ist auch kein Kontaminations-Kontroll-System vorhanden.

In folgenden Literaturstellen werden noch alternative Verfahren für eine Amplifizierung von Nukleinsäuren bzw. Nukleinsäuresequenzen mittels spezieller Enzymsysteme (Restriktionsendonukleasen bzw. Q-beta Replikasen) beschrieben:

G.T. Walker et al., Proc. Natl. Acad. Sci. (USA) 89, 392-6 (1992); US-PS 5356774 (V.D. Axelrod et al.); und P. Knight, Bio/Technology 7, 609-10 (1989).

WO 90/10064 and WO 91/03573 beschreiben die Verwendung des Bakteriophagen phi29-Replikationsursprungs für die isotherme Amplifizierung von Nukleinsäuren.

Die sogenannte Strang-Verdrängungsamplifizierung (US-PS 5455166 und US-PS 5422252) beruht auf der Strangverdrängungsaktivität von Polymerasen, die an bestimmten, durch Primersequenzen vorgegebenen Stellen und durch spezielle Endonukleasen im Reaktionsgemisch erzeugten Startpunkten einen der beiden komplementären Nukleinsäurestränge vom anderen löst und gleichzeitig einen neuen komplementären Strang synthetisiert.

Ein einfacheres isothermes Strang-Verdrängungs-Amplifizierungs-Verfahren wird in EP-A 0676476 beschrieben. In diesem Verfahren ist lediglich eine Polymerase als Enzym notwendig, allerdings sind mindestens zwei Oligonukleotide pro Strang, also insgesamt mindestens vier Oligonukleotide für die Amplifizierung nötig.

Eine der Sonderformen der PCR stellt die sogenannte "Nested"-PCR (z.B. US-PS 5340728, EP-A 0519338, US-PS 4683195, US-PS 5314809) dar.

Eine weitere Sonderform der PCR stellt die sogenannte RT-PCR dar, wobei RNA-Template zuerst in cDNA transkribiert wird. Dabei kann ein Enzym verwendet werden, das sowohl Reverse Transkriptase-Aktivität, als auch DNA-Polymerase-Aktivität hat (z.B. US-PS 5322770).

Obwohl alle diese Methoden ihren Platz in der Forschung gefunden haben - die PCR ist aus einem modernen molekularbiologischen Labor nicht mehr wegzudenken - sind sie dennoch komplex und stellen so hohe Anforderungen an Gerätschaft und Personal, daß sie kaum als Routinemethoden bezeichnet werden können. Ebenso sind mit diesen Methoden hohe Geräte- und Reagenzkosten verbunden, da insbesondere für das Temperatur-Cycling teure Hochleistungsthermostaten nötig sind (vgl. R. Hoelzel, "Trends in Genetics", 6, 237-238, 1990; oder U. Linz, Biotechniques, 9, 286-292, 1990) und andererseits für die isothermen Methoden teure Enzyme verwendet werden müssen.

Die WO 94/03635 betrifft Amplifikations- und Detektionsverfahren für Nukleinsäuren. Gemäß der Offenbarung kann dieses Amplifikationsverfahren alternativ zur Verwendung von zwei verschiedenen Temperaturen auch isotherm gefahren werden. Allgemein ist das Amplifikationsverfahren jedoch auf die Verwendung von bestimmten Primern beschränkt, welche derart ausgewählt werden, daß sie an die Stränge der Targetsequenz stark binden, die Extensionsprodukte von jedem Primer jedoch nur schwach an den Strang binden. Dies wird dadurch erreicht, daß die Primer derartig ausgewählt werden, daß sie ein bestimmtes Verhältnis von G und C zu A und T aufweisen, welches von etwa 1,5:1 bis etwa 3,0:1 reicht.

Erst die Verwendung derartiger Primer ermöglicht eine isotherme Führung des Verfahrens. Bei dem einzigen Beispiel für eine isotherme Amplifikationsreaktion handelt es sich jedoch aufgrund der verwendeten Polymerase um eine lineare Amplifikation.

Die einzige Offenbarung eines isothermen Amplifikationsverfahrens gemäß Beispiel 2 führt somit vom Gegenstand der vorliegenden Erfindung weg, da einem Fachmann bei genauem Studium der angeführten Reaktionsbedingungen und verwendeten Reagenzien klar ist, daß das Beispiel nicht funktionieren kann. Auch das Weglassen des offensichtlich falschen Einsatzes von dUTP (welches von der verwendeten Polymerase nicht gelesen werden kann) führt nicht zu einer exponentiellen isothermen Amplifikationsreaktion, sondern lediglich zu einer linearen Amplifizierung, und dies auch nur bei Verwendung eines spezifischen Primer-Paares.

Die WO 95/25180 wurde erst am 21. September 1995, also nach dem Prioritätsdatum der vorliegenden Anmeldung, veröffentlicht.

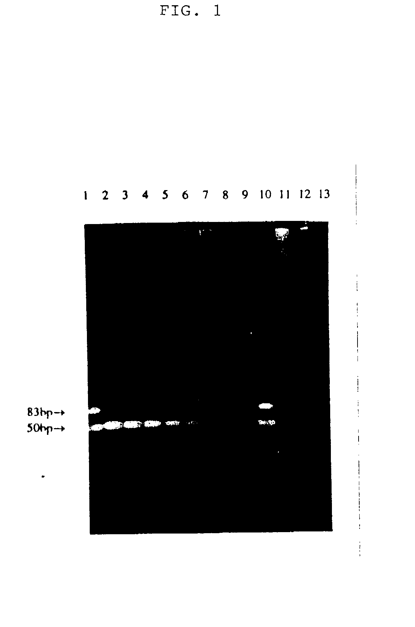

Bei Studium der Offenbarung der WO 95/25180 sind zwei Fälle zu unterscheiden. Wenn von einem Einzelstrang ausgegangen wird, muß ein "Set" von Primern verwendet werden, welches "Set" zumindest zwei zur Targetnukleinsäure komplementäre Primer enthält. Demgemäß sind also pro Strang zumindest zwei zur Zielsequenz komplementäre Primer zur Durchführung der Amplifikationsreaktion nötig. Weiters ist zu beachten, daß, wenn von einem Einzelstrang ausgegangen wird und lediglich ein Set von Primern verwendet wird, welches zum Strang der Zielsequenz komplementär ist, nur eine lineare Amplifikation erreicht werden kann, da die Primer immer nur auf das ursprüngliche Templat hybridisieren können und die Menge an Templat dadurch nicht vermehrt werden kann, was eine Grundvoraussetzung einer exponentiellen Amplifikation darstellt. Wenn nun ein Doppelstrang zu amplifizieren ist, wobei Primer und "sense"-Primer einzusetzen sind, d.h. wie aus Fig. 1 ersichtlich jeweils ein Set Primer pro Strang, umfaßt dieser Primer "array" mindestens zwei Sets von Primern, also mindestens vier Primer. In solch einem Fall, wenn also zu jedem Strang des Doppelstranges mindestens zwei komplementäre Primer verwendet werden, ist eine exponentielle Amplifikation zwar möglich, eine Vielzahl von Primern (es werden bis zu zwanzig Primer und sense-Primer verwendet), kompliziert natürlich ein Verfahren, während erfindungsgemäß überraschenderweise gefunden wurde, daß ein Primer pro Strang des Doppelstranges ausreicht bzw. ein Maximum von drei Primern zweckmäßig ist.

### Definitionen der in Beschreibung und Patentansprüche verwendeten Begriffe:

Isotherm: Unter "im wesentlichen isotherm" bzw. "bei im wesentlichen derselben Temperatur" ist eine Abweichung von der vorgegebenen Temperatur im Bereich der Abweichung eines handelsüblilichen Thermostaten zu verstehen.

Ausgangsmaterial: Beim erfindungsgemäßen Verfahren kann jede Nukleinsäure bzw. Nukleinsäuresequenz in gereinigter oder ungereinigter Form als Ausgangsmaterial verwendet werden, vorausgesetzt, es enthält (bzw. es wird angenommen, daß es enthält) im wesentlichen die für die verwendeten Oligonukleotidbausteine spezifische Nukleinsäuresequenz. Für das ertindungsgemäße Verfahren kann DNA oder RNA (inklusive mRNA, rRNA, tRNA, etc.) in einzelsträngiger oder doppelsträngiger Form verwendet werden. Darüberhinaus kann auch ein DNA-RNA-Hybrid als Template verwendet werden. Eine Mischung dieser Nukleinsäurespezies kann ebenso verwendet werden wie Nukleinsäure aus vorhergehenden Amplifikationen, und zwar mit gleichen oder unterschiedlichen Oligonukleotiden wie in der ersten Amplifikation.

Die zu amplifizierende spezifische Nukleinsäure bzw. Nukleinsäuresequenz kann ein Teil eines größeren Moleküls sein oder kann als Molekül schon in der Form vorhanden sein, daß die spezifische Sequenz der ganzen Nukleinsäure entspricht.

Es ist nicht notwendig, daß die zu amplifizierende Sequenz in reiner Form vorliegt, sie kann auch nur als Bruchteil einer komplexen Mischung vorliegen (z.B. als Teil eines Genoms). Dieses Genom selbst kann auch nur ein Teil einer biologischen Probe sein (z.B. Zellen oder Gewebe).

Die Ausgangsnukleinsäure bzw. -sequenz kann eine oder mehrere spezifische Nukleinsäuresubsequenzen enthalten (die gleich oder unterschiedlich sein können). Die Amplifikationsmethode ist daher nicht nur dafür geeignet, große Mengen einer spezifischen Nukleinsäure herzustellen, sondern auch um gleichzeitig mehrere Subsequenzen der Ausgangsnukleinsäure bzw. -sequenz in einem Reaktionsgefäß zu amplifizieren.

Die gegebenenfalls vor Reaktionsbeginn in Einzelstränge aufgetrennte ("denaturierte") Ausgangsnukleinsäure bzw. -sequenz, kann aus verschiedenen Quellen stammen, z.B. von Plasmiden wie pBR322, von klonierter DNA oder RNA, von chemisch synthetisierter DNA oder RNA, aus natürlicher DNA oder RNA beliebiger Herkunft (z.B. aus Bakterien, Hefen, Pilzen, Viren und Organellen, oder aus höheren Organismen wie z.B. Pflanzen, Tieren oder Menschen). DNA oder RNA kann z.B. aus Blut, Körperflüssigkeiten, Pflanzensäften, Nährmedien oder Geweben mit einer Vielzahl von Techniken extrahiert werden - vgl. z.B. "Molecular Cloning. A Laboratory Manual", J. Sambrook et al., Cold Spring Harbor Laboratory Press, New York (1989) - es können aber auch nicht extrahierte Gewebeteile oder Zellen verwendet werden. Grundsätzlich kann durch das Verfahren der vorliegenden Erfindung jede spezifische NukleinsäureSequenz amplifiziert werden.

Chemische Bausteine: Unter "chemische Bausteine" werden in der vorliegenden Schrift solche verstanden, die zur enzymatischen Synthese von Nukleinsäuren verwendet werden bzw, verwendet werden können, wie z.B. Deoxyribonukleotidtriphosphate, Ribonukleotidtriphosphate, Oligonukleotide (wie Ribooligonukleotide oder Deoxyribooligonukleotide). Es können aber auch modifizierte Bausteine verwendet werden, etwa solche, die bestimmte Markierungen, wie Biotin und dergl., oder Reaktionsgruppen, beispielsweise Phosphatgruppen, tragen. Denkbar sind auch kleinere Bausteine, wie z.B. Teile von Nukleotiden und Nukleosiden und Derivate davon.

Oligonukleotidbausteine: Die Bezeichnung "Oligonukleotid" wird hier für natürliche oder synthetische Moleküle verwendet, die aus mindestens zwei Deoxyribonukleotiden oder Ribonukleotiden bzw. Derivaten hievon bestehen. Die Veränderung kann sowohl den Basenteil als auch das Rückgrat der Oligonukleotide betreffen (z.B. Peptid-Nukleinsäuren, Hexose-Phosphate, etc.)

Die genaue Größe der Oligonukleotide hängt von mehreren Faktoren ab, welche ihrerseits wieder von der jeweiligen Verwendung oder Funktion des Oligonukleotids abhängen. Oligonukleotide können chemisch synthetisch oder durch Klonieren oder Präparieren von DNA- oder RNA-Sequenzen hergestellt werden.

Oligonukleotidbausteine sind Oligonukleotide die als chemische Bausteine in der vorliegenden Erfindung verwendet werden. Ein Oligonukleotidbaustein ist also eine Nukleinsäure, eine Nukleinsäuresequenz oder ein Oligonukleotid, welche unter geeigneten Bedingungen (d.h. unter Einfluß von Enzymen, Anwesenheit der erforderlichen chemischen Bausteine, wie z.B. weitere Oligonukleotidbausteine, Nukleotidtriphosphaten, Pufferbedingungen wie z.B. pH, Ionenstärke, Kofaktoren, etc.) und bei passender Temperatur als chemischer Baustein für die Nukleinsäure-Synthese dienen kann.

Hinsichtlich der Übereinstimmung der komplementären Basensequenzen von Oligonukleotidbausteinen und Template ist lediglich erforderlich, daß eine ausreichende Zahl von Basen an beiden Enden der spezifischen und zu amplifizierenden NukleinsäureSequenz im wesentlichen bekannt sind, sodaß als Oligonukleotidbausteine mindestens zwei Nukleinsäuren hergestellt werden können, von denen mindestens eine an den jeweiligen entgegengesetzten Nukleinsäure-Strängen hybridisieren können. Die relative Position auf der Sequenz wird dabei vorzugsweise so gewählt, daß das Produkt, das von einem Oligonukleotidbaustein (bei Polymerase-katalysierten Amplifikationen) bzw. von zwei Oligonukleotidbausteinen (für Ligase-katalysierte Reaktionen) gebildet wird, als Template für die enzymatische "Verlängerung" (für Polymerase katalysierte Reaktionen) des anderen Oligonukleotidbausteins bzw. für die "Verknüpfung" (für Ligase katalysierte Reaktionen) der anderen Oligonukleotidbausteine dienen kann.

Oligonukleotidbausteine können aus natürlichen Quellen stammen, wie z.B. restriktionsverdaute DNA, oder chemisch synthetisch und/oder enzymatisch hergestellt werden. Die genaue Länge der Oligonukleotidbausteine hängt von mehreren Faktoren ab, wie z.B. Temperatur, Templatesequenz, etc. Im Allgemeinen sind Oligonukleotidbausteine zwischen 15 und 40 Basen lang, können aber auch kürzer oder länger sein. Kürzere Oligonukleotidbausteine benötigen in der Regel niedrigere Temperaturen für eine effiziente Reaktion. Die im erfindungsgemäßen Verfahren verwendeten Oligonukleotidbausteine müssen jedenfalls zu den jeweiligen spezifischen Sequenzen, die amplifiziert werden sollen, im wesentlichen komplementär sein.

Ein Oligonukleotidbaustein muß also nicht exakt komplementär zum jeweiligen Template-Strang sein. Es ist z.B. möglich, nicht-komplementäre Nukleotidsequenzen und/oder Markierungen ( wie z.B. Haptene oder Enzyme) an den äußeren Enden der zu reagierenden Oligonukleotidbausteine anzuhängen.

Transkription: "Transkription" ist der Prozeß der Bildung eines RNA-Stranges unter Verwendung eines DNA-Stranges als Matrize. Ein Sonderfall der Transkription ist die Reverse Transkription, bei der ein DNA-Strang unter Verwendung eines RNA-Stranges als Matrize hergestellt (gebildet) wird. Unter "Transkription" wird in der vorliegenden Beschreibung jedenfalls ein Prozeß verstanden, der von einer Nukleinsäureart (wie RNA oder DNA) zur jeweils anderen Nukleinsäureart (DNA oder RNA) führt.

Template: Jede Nukleinsäure bzw. Nukleinsäuresequenz, die als Basis für die Synthese der entsprechenden komplementären Nukleinsäurestränge dienen kann, wird als Template bezeichnet. Sowohl die Ausgangsnukleinsäure als auch die Zwischenprodukte und Reaktionsprodukte können Template sein.

Mutation: Eine "Mutation" ist eine Veränderung in der Basenzusammensetzung einer Nukleinsäure, wobei die vorhandenen Basen den natürlichen entsprechen können und als Unterschied das Fehlen, der Austausch durch andere oder das Einfügen von Basen zu sehen ist. Es können aber auch die Zusammensetzungen der Nukleinsäuren relativ gleich sein und die Veränderung sich auf eine chemische Modifikation des Moleküls beziehen. Es kann aber auch eine Kombination der oben erwähnten Arten gemeint sein.

Enzyme: Enzyme sind üblicherweise Polypeptide, die eine chemische Reaktion katalysieren. Es können aber enzymatische Aktivitäten auch bei anderen Stoffklassen gefunden und als Ezyme verwendet werden (z.B. RNA, synthetische Polymere). Unter Enzym wird in der vorliegenden Schrift auch ein Gemisch von verschiedenen Enzymen mit ähnlichen Eigenschaften verstanden.

Hilfsoligonukleotide: Hilfsoligonukleotide sind Oligonukleotide, die eine chemische Reaktion verbessern, ohne als chemischer Baustein in dieser Reaktion verbraucht zu werden.

Reaktionsprodukt: Das Reaktionsprodukt ist ein doppelsträngiges Nukleinsäuremolekül, das aus den zugesetzen chemischen Bausteinen durch enzymatische Aktivität erzeugt wurde und zum Teil auch aus Template bestehen kann.

### Zusammenfassung der Erfindung:

Die vorliegende Erfindung betrifft ein Verfahren zur transkriptionsfreien exponentiellen Amplifizierung von Nukleinsäuren bzw. Nukleinsäuresequenzen mittels Enzymen, wobei die Nukleinsäuren bzw. Nukleinsäuresequenzen gegebenenfalls vor der Amplifizierung zumindest teilweise in ihre Einzelstränge aufgetrennt und/oder transkribiert werden, und in der Reaktionsmischung Oligonukleotide mit einer Basensequenz im wesentlichen komplementär zu den Basensequenzen der Enden der zu amplifizierenden Nukleinsäure bzw. Nukleinsäuresequenz enthalten sind, welche Oligonukleotide unter geeigneten Bedingungen Startpunkte und/oder chemische Bausteine zur Nukleinsäuresynthese bilden können und in das zu bildende Amplifizierungsprodukt eingebaut werden, wobei das Produkt aus den Bausteinen selbst wieder der zu amplifizierenden Nukleinsäure bzw. Nukleinsäuresequenz entspricht, dadurch gekennzeichnet, daß
- das Reaktionsprodukt, welches aus den Oligonukleotidbausteinen, gegebenenfalls dem Template und gegebenenfalls aus weiteren chemischen Bausteinen gebildet wurde, in einem Schritt wieder mit den Oligonukleotidbausteinen reagiert,
- daß die Reaktion bei einer die Doppelstrangform des Reaktionsproduktes destabilisierenden Temperatur im wesentlichen isotherm geführt wird und
- daß bei Verwendung von Polymerase als Enzym diese Strangverdrängungsaktivität aufweist.

### Detaillierte Beschreibung der Erfindung:

Aufgabe der vorliegenden Erfindung ist es, die bekannten Nachteile der herkömmlichen Verfahren wie PCR, LCR oder 3SR zu überwinden und ein einfaches, kostengünstiges Verfahren der eingangs angegebenen Art vorzusehen.

Diese Aufgabe wird erfindungsgemäß dadurch gelöst, daß das Verfahren bei einer die Doppelstrangform des Reaktionsproduktes destabilisierenden Temperatur im wesentlichen isotherm abläuft und nur ein Enzym verwendet werden muß.

Bei einer derartigen isothermen Verfahrensführung findet die Amplifizierung unter Beibehaltung der Doppelstrangform der Nukleinsäure bzw. Nukleinsäurefragmente, d.h. unter weitgehender Beibehaltung von Wasserstoffbrücken zwischen den Basenpaaren von komplementären, zu amplifizierenden Nukleinsäuren bzw. Nukleinsäurefragmenten statt. Mit anderen Worten bedeutet dies, daß während der Amplifizierung komplementäre zu amplifizierende Nukleinsäuren bzw. Nukleinsäurefragmente als Doppelstränge vorliegen.

Alle Reaktanden, die Nukleotidtriphosphate, Oligonukleotidbausteine, das Produkt, das Template und das Enzym stehen untereinander sowie mit allen anderen Reaktionskomponenten im Fließgleichgewicht. Die Reaktionsbedingungen und insbesondere die Reaktionstemperatur werden dabei erfindungsgemäß derart gewählt, daß auch das Amplifikationsprodukt bevorzugt in doppelsträngiger Form vorliegt. Obwohl der Reaktionsmechanismus noch nicht endgültig geklärt werden konnte, wird aufgrund der vorliegenden Daten und Versuchsergebnisse angenommen, daß beim erfindungsgemäßen Verfahren die zugesetzten Oligonukleotidbausteine im Widerspruch zur gängigen Auffassung über derartige molekulare Mechanismen bereits an der Doppelstrangform der eingesetzten Nukleinsäure bzw. Nukleinsäurefragmente angreifen und somit die bei herkömmlichen Verfahren wie PCR, LCR, 3SR, etc. zwingend notwendige Auftrennung von Nukleinsäure oder Nukleinsäurefragmenten - bzw. im weiteren Reaktionsverlauf der Reaktionszwischenprodukte in die Einzelstränge nicht erforderlich ist.

Gängige Verfahren beruhen darauf, daß die Reaktionszwischenprodukte in ihre Einzelstränge getrennt werden und dann die Oligonukleotide bevorzugt mit den Einzelsträngen hybridisieren. Das erfindungsgemäße Verfahren beruht hingegen auf der überraschenden Erkenntnis, daß die Oligonukleotide bei gegebener Temperatur in einem Schritt mit dem Ausgangsmaterial bzw. dem Amplifikationsprodukt reagieren und aus diesen Reaktionsprodukten unter enzymatischer Katalyse wiederum Amplifikationsprodukte enstehen. Hierbei besteht zu den Verfahren des Standes der Technik der wesentliche und prinzipielle Unterschied, daß in der PCR bzw. in der LCR sowie in verwandten Verfahren den Oligonukleotiden durch Hitzedenaturierung Einzelstränge als Templates vorbereitet werden, an welche die Primer und Oligonukleotidbausteine dann hybridisieren. Beim erfindungsgemäßen Verfahren wird hingegen die Tatsache ausgenutzt, daß bei Vorliegen dynamischer Gleichgewichte die Reaktionsprodukte durch Zugabe eines Reaktionspartners im Überschuß favorisiert werden können. Diese Art der Reaktion wird Strang Invasion (bzw. Strang-Austausch) bezeichnet und wurde nach dem Anmeldetag der Prioritätsanmeldung zur vorliegenden Erfindung an sogenannter D-Form DNA nachgewiesen (Iyer, M. et al. J. Biol. Chem. 270, 14712, 1995). Die Strang-Invasion konnte jedoch nicht bei B-Form DNA, der häufigsten Form, nachgewiesen werden. In der vorliegenden Erfindung wird die B-Form Nukleinsäurehelix durch Hitze destabilisiert, sodaß sie überraschenderweise einer Strangaustausch-Reaktion zugänglich ist. Es sind auch Peptide beschrieben, die, eine Strangaustausch-Reaktion verbessern (Corey, D.R., J. Am. Chem. Soc., 9373, 1995). Derartige Peptidsequenzen könnten auch in der vorliegenden Erfindung verwendet werden. Ebenfalls denkbar ist der Einsatz sogenannter Peptidnukleinsäuren, um die Strangaustausch-Reaktion zu verbessern (Iyer, M. et al. J. Biol. Chem. 270, 14712,1995).

Somit werden beim erfindungsgemäßen Verfahren den Oligonukleotidbausteinen keine Einzelstränge als Templates angeboten, vielmehr reagieren die Oligonukleotidbausteine bei einem isothermen Reaktionsverlauf von sich aus mit dem doppelsträngigen Amplifizierungsprodukt und bilden neue, unvollständige Reaktionspartner, die wiederum sofort mit den jeweiligen Enzymen und sonstigen Reaktionspartnern autokatalytisch reagieren können, um so neue Amplifizierungsprodukte zu bilden.

Für das erfindungsgemäße Verfahren können verschiedene Enzyme einzeln oder in Kombination verwendet werden. Werden Polymerasen verwendet, beginnt die Synthese am 3'-Ende des Oligonukleotidbausteins und wird in Richtung 5'-Ende entlang dem Template fortgesetzt. Es sind aber auch Enzyme denkbar, die die Synthese am 5'-Ende des Primers starten und in Richtung 3'-Ende fortsetzen. Auch solche Enzyme könnten für das erfindungsgemäße Verfahren verwendet werden. Weiters ist denkbar, daß eine Oligonukleotidbaustein-abhängige RNA-Polymerase als Enzym verwendet wird. Wird Ligase eingesetzt, werden im Allgemeinen teilweise Oligonukleotidbausteine verwendet, die an den Enden Phosphatgruppen tragen. Es ist auch denkbar, eine Mischung von ähnlichen Enzymen, oder verschiedenen Enzymen in einer Reaktion zu verwenden, um bestimmte Amplifizierungen zu erreichen (z.B. zwei oder mehrere verschiedene Polymerasen oder Polymerasen und Ligasen). Die spezifische Nukleinsäure oder - sequenz wird unter Verwendung einer nukleinsäurehältigen Probe, die eben diese Sequenz (oder einen Teil davon) als Template enthält, produziert. Wenn die herzustellende Nukleinsäure bzw. Nukleinsäuresequenz DNA ist, sind bei Verwendung von Polymerase die vier vorzugsweise zu verwendenden Nukleotidtriphosphate dATP, dCTP, dGTP und TTP.

Die für die Reaktion notwendige Oligonukleotidkonzentration kann variieren, liegt jedoch im allgemeinen über der Template-Konzentration (in molaren Verhältnissen ausgedrückt). Ein großer molarer Überschuß verbessert normalerweise die Reaktion.

Ein vorbereitender Schritt des erfindungsgemäßen Verfahrens besteht gegebenenfalls in der Denaturierung (Strangtrennung) bzw. der Transkription der Nukleinsäure, sodaß bei anschließendem Abkühlen auf die Reaktionstemperatur intensiver Kontakt zwischen den Template-Strängen, den Oligonukleotidbausteinen, ggf. den Nukleotidtriphosphaten und den entsprechenden Enzymen - vorzugsweise Polymerase oder Ligase - möglich ist. Sofern noch nicht in der Reaktionslösung vorhanden, werden anschließend das gewählte Enzym und die benötigten chemischen Bausteine zugegeben und das Reaktionsgemisch auf passende Reaktionstemperatur gebracht. Die Reaktionsbedingungen und insbesondere die Reaktionstemperatur werden dabei so gewählt, daß die Amplifikation unter Ausnutzung des dynamischen Gleichgewichtes zwischen Amplifizierungsprodukt und chemischen Bausteinen ohne thermische Strangauftrennung möglich ist, wobei angenommen wird, daß die verwendeten Oligonukleotide direkt in die Doppelstrangform des Amplifizierungsproduktes an ihre komplementären Bindungsstellen "hineinhybridisieren" und dabei die Doppelstrangform des Amplifizierungsproduktes reißverschlußartig aufgeht.

Die spezielle Reaktionstemperatur wird üblicherweise aus Gründen der Spezifität höher als die errechnete Schmelztemperatur der Oligonukleotidbausteine gewählt.

Die Schmelztemperatur der Oligonukleotidbausteine kann nach verschiedenen Methoden errechnet werden, die optimale Amplifizierungstemperatur ist jedoch darüber hinaus auch noch von vielen anderen Faktoren (wie z.B. Ionenstärke des Puffers, Konzentration der Oligonukleotidbausteine, Länge und Basenzusammensetzung des Templates, etc.) abhängig.

Vorteilhaft ist auch, wenn die Oligonukleotidbausteine am Template möglichst nahe beisammen liegen, am besten so, daß das Amplifizierungsprodukt gleich groß oder nicht wesentlich größer ist als die Summe der Länge der eingesetzten Oligonukleotide.

Zur Durchführung der vorliegenden Erfindung werden Reagenzkits verwendet, welche Oligonukleotidbausteine zur Verwendung in dem erfindungsgemäßen Verfahren enthalten, wobei die Differenz der Schmelztemperaturen zwischen den Oligonukleotidbausteinen und dem erwünschten Produkt nicht mehr als 25 °C, vorzugsweise nicht mehr als 20°C und insbesondere nicht mehr als 15 °C beträgt.

Besonders bevorzugt wird hiebei, wenn die Differenz der Schmelztemperaturen zwischen den Oligonukleotidbausteinen und dem erwünschten Produkt nicht mehr als 10°C, vorzugsweise nicht mehr als 5 °C beträgt.

Für diagnostische Methoden ist es am günstigsten, wenn die Basensequenz der Oligonukleotidbausteine zumindest zu einem Teil der Targetsequenz exakt komplementär ist.

Die Vorteile des erfindungsgemäßen Verfahrens bestehen insbesondere darin, daß keine Temperaturzyklen eingehalten werden müssen, d.h. daß auch die Notwendigkeit für eine teure Instrumentierung entfällt, daß einfacher standardisiert werden kann, große Reaktionsansätze (1 ml und mehr) durchgeführt werden können und schließlich, daß lediglich ein einziges Enzym benötigt wird. Selbstverständlich kann das erfindungsgemäße Verfahren auch im Zuge gängiger Verfahren wie PCR bzw. LCR sowie in verwandten Verfahren zum Einsatz kommen. Vorzugsweise liegt dabei die Temperatur des Amplifizierungsschrittes über der errechneten Schmelztemperatur der verwendeten Oligonukleotide.

Es sind viele Sonderformen der vorliegenden Erfindung denkbar, z.B. nested, d.h. daß zuerst mit äußeren Oligonukleotidbausteinen amplifiziert wird, danach Teile des Amplifizierungsprodukts weiteramplifiziert werden. Auch eine Kopplung der Amplifizierung mit einer Reversen Transkription in einem Reaktionsansatz ist denkbar.

Der Nachweis der Amplifizierungsprodukte erfolgt prinzipiell gleich wie bei der PCR. Besonders günstig erscheinen Fluoreszenzmethoden, die auf Energy Transfer (Förster, T., Discuss. Faraday Soc., 27, 7, 1959) oder Fluoreszenzpolarisation von einem oder zwei markierten Oligonukleotidbausteinen beruhen. Denkbar sind auch Fluoreszenzmethoden, bei denen während der Amplifzierung markierte chemische Bausteine in das Produkt eingebaut werden und dieser Einbau fluorimetrisch oder luminometrisch detektiert wird.

Das erfindungsgemäße Verfahren wird nun anhand einiger Beispiele näher erläutert, ohne jedoch darauf beschränkt zu sein.

### Beispiel 1: Vermehrung einer linearen, einzelsträngigen DNA

Dieses Beispiel zeigt, daß es möglich ist, ein bestimmtes einzelsträngiges DNA-Molekl in vitro enzymatisch zu vermehren. Dabei wird mit zwei Oligonukleotidbausteinen, die an zwei komplementäre DNA-Stränge hybridisieren, ein doppelsträngiges Produkt gebildet, wobei auf das herkömmliche wiederholte Wechseln der Reaktionstemperatur zwischen Amplifizierungs- und Denaturierungstemperatur völlig verzichtet wird. Alle Arbeitstechniken und Methoden, die nicht näher beschrieben werden, sind Standardtechniken, die in Methodensammlungen für die Gentechnik oder Molekularbiologie beschrieben sind (wie z.B. in "Molecular Cloning. A laboratory manual"; Sambrook et al.; Cold Spring Harbor Laboratory Press, New York 1989; ISBN 0-87969-309-6)

### 1.1. Reaktionsansatz:

Es wurden jeweils 50 µl Reaktionsansätze in 0,5 ml Reaktionsgefäßen (Eppendorf, Safe-Lock) zusammenpipettiert. Die Reaktionsansätze wiesen dabei folgende Zusammensetzung auf:
10 mM (NH₄)₂SO₄
20 mM Tris-HCl (pH 8,8 bei 25 °C)
2 mM MgSO₄
je 200 µM TTP, dGTP, dATP und dCTP (von PROMEGA, Wisc., USA)
0,1% (M/V) Triton X-100 (von SERVA, Deutschland)
10 pmol Oligonukleotidbaustein u676: 5'-dgAgCCTTCAACCCAgTCAgCTCCTTCCggTgggCgCggggC-3'
10 pmol Oligonukleotidbaustein 1755: 5'-dCgCCgAAAATgACCCAgAgCgCTgCCggCACCTgTCCTACgAgTTgCATg-3'
1 fmol Template-DNA:

5'-dgAgCCTTCAACCCAgTCAgCTCCTTCCggTgggCgCggggCCATgCAACTCg-
TAggACAggTgCCggCAgCgCTCTgggTCATTTTCggCg-3'

Die Oligonukleotide und das DNA-Template wurden von CODON genetic systems (Weiden/See, AT) erworben.

Als Negativkontrollen dienten Reaktionsansätze, denen keine Template-DNA zugesetzt wurde.

Anschließend wurden die Reaktionsansätze mit 100 µl Mineralöl (SIGMA, M-3516) überschichtet und auf einem Perkin Elmer Cetus GeneAmp PCR System 9600 Thermostatisiergerät auf 84 °C erwärmt. Zwei Units des Enzyms Deep Vent_{R}(exo⁻) DNA Polymerase (New England Biolabs, USA) wurden dem Gemisch nach Erreichen der Reaktionstemperatur zugesetzt.

Anschließend wurden einzelne Proben und Negativkontrollen bei 84 **°**C unterschiedlich lang weiterinkubiert, um einen Verlauf der Amplifizierung feststellen zu können. 10 µl der Reaktionsgemische wurden auf einem 2%igen Agarosegel aufgetrennt und durch Färbung mit Ethidiumbromid und UV-Fluoreszenzanalyse sichtbar gemacht. Das Amplifizierungsprodukt konnte durch Vergleich mit einem DNA-Längenstandard als einzige Bande identifiziert werden.

### 1.2. Zeitverlauf der Amplifizierung:

Die Produkte der einzelnen Reaktionsanstze wurden nach der Elektrophorese quantifiziert. Die Quantifizierung erfolgte durch serielle Verdünnung der einzelnen Reaktionsgemische und Vergleich der Verdünnungsstufen mit einem DNA-Standard und untereinander.

Die Ergebnisse dieser Analyse sind in Tabelle 1 zusammengefaßt.

### 1.3. Molekulare Klonierung und Sequenzierung eines Amplifizierungsproduktes

Nach der Amplifikation wurde das Reaktionsgemisch mit Phenol/Chloroform und Chloroform extrahiert und die DNA mit Ethanol ausgefällt. Die getrocknete DNA wurde in Phosphorylierungspuffer (Boehringer Mannheim) mit 1 mM ATP und 10 Einheiten des Enzyms T4 Polynucleotid Kinase (Boehringer Mannheim) in einem Endvolumen von 10 µl 1 Stunde bei 37°C inkubiert. Anschließend wird dem Ansatz 1,5 µl steriles destilliertes Wasser, 1,5 µl 1M MgCl₂, 1 µl 1mM dNTP (je 250 µM TTP, dGTP, dCTP, dATP) und 1 µl (= 2 Einheiten) Klenow-Enzym zugesetzt und eine weitere Stunde bei 37°C inkubiert.

Das Reaktionsgemisch wurde auf einem präparativen Agarosegel aufgetrennt, die DNA-Bande aus dem Gel gereinigt und zur Ligation verwendet. Die Ligation des Produktes in einen mit SmaI geschnittenen und dephosphorylierten Plasmidvektor (p Bluescript IISK+; Stratagene) erfolgte nach Standardverfahren. Nach der Transformation in kompetente Bakterien *Escherichia coli* MC1061 wurden einige Bakterienkolonien zur Herstellung von Plasmid-DNA für die Sequenzierung benutzt.

Die Sequenzierung des klonierten Produktes mit den Primern M13 sequencing und M13 reverse sequencing (CODON genetic systems) erfolgte nach der Dye Deoxy Terminator Methode von Applied Biosystems mit dem Sequenziergerät ABI 373A.

Die Sequenz des Amplifizierungsproduktes lautete:

5'-dgAgCCTTCAACCCAgTCAgCTCCTTCCggTgggCgCggggCCATgCAACTCg
TAggACAggTgCCggCAgCgCTCTgggTCATTTTCggCg-3' (Plus Strang)
und
3'-dCTCggAAgTTgggTCAgTCgAggAAggCCACCCgCgCCCCggTACgTTgAgCA
TCCTgTCCACggCCgTCgCgAgACCCAgTAAAAgCCgC-5' (Minus Strang)

Damit wurde gezeigt, daß das Amplifizierungsprodukt genau der durch die beiden Oligonukleotidbausteine definierten Sequenz entspricht

**Tabelle 1:**

| Verlauf der Zunahme des Reaktionsproduktes | |
|---|---|
| Reaktionszeit in Minuten | Produktmenge in pmol |
| 0 | 0,00 |
| 20 | 0,24 |
| 40 | 0,48 |
| 60 | 1,44 |
| 120 | 4,33 |
| 240 | 8,65 |

### Beispiel 2: Vermehrung einer linearen, doppelsträngigen DNA

Dieses Beispiel zeigt, daß es möglich ist, ein bestimmtes doppelsträngiges DNA-Molekül in vitro enzymatisch ohne Denaturierungsschritt zu vermehren.

Dabei wird mit zwei Oligonukleotiden, die an zwei komplementäre DNA-Stränge hybridisieren, ein doppelsträngiges Produkt gebildet, wobei auf das herkömmliche wiederholte Wechseln der Reaktionstemperatur zwischen Amplifizierungs- und Denaturierungstemperatur völlig verzichtet wird.

### 2.1. Reaktionsansatz:

Es wurden jeweils 50 µl Reaktionsansätze in 0,5 ml Reaktionsgefäßen (Eppendorf, Safe-Lock) zusammenpipettiert. Die Reaktionsansätze wiesen dabei folgende Zusammensetzung auf:
10 mM (NH₄)₂SO₄
20 mM Tris-HCl (pH 8,8 bei 25 °C)
2 mM MgSO₄
je 200 µM TTP, dGTP, dATP, dCTP
0,1% (M/V) Triton X-100
10 pmol Oligonukleotidbaustein u676: (5'-dgAgCCTTCAACCCAgTCAgCTCCTTCCggTgggCgCggggC-3')
10 pmol Oligonukleotidbaustein 1755 
(5'-dCgCCgAAAATgACCCAgAgCgCTgCCggCACCTgTCCTACgAgTTgCATg-
3')
1 fmol Template-DNA:

5'-dgAgCCTTCAACCCAgTCAgCTCCTTCCggTgggCgCggggCCATgCAACTCg
TAggACAggTgCCggCAgCgCTCTgggTCATTTTCggCg-3'

3'-dCTCggAAgTTgggTCAgTCgAggAAggCCACCCgCgCCCCggTACgTTgAgCA
TCCTgTCCACggCCgTCgCgAgACCCAgTAAAAgCCgC-5'

Als Negativkontrollen dienten Reaktionsansätze, denen keine Template-DNA zugesetzt wurde.

Anschließend wurden die Reaktionsansätze mit je 100 µl Mineralöl (SIGMA, M-3516) überschichtet und auf einem Perkin Elmer Cetus Gene Amp PCR System 9600 Thermostatisiergerät auf 84 °C erwärmt. Nach Erreichen der Reaktionstemperatur wurden dem Gemisch 2 Units des Enzyms Deep Vent_{R}(exo⁻) DNA Polymerase (New England Biolabs) zugesetzt. Anschließend wurden Proben und Negativkontrollen bei 84° C 14 Stunden lang weiterinkubiert.

### 2.2. Visualisierung:

Je 10 µl der Reaktionsgemische wurden auf einem 2%igen Agarosegel aufgetrennt und durch Färbung mit Ethidiumbromid und UV Fluoreszenzanalyse sichtbar gemacht. Das Amplifizierungsprodukt konnte durch Vergleich mit einem DNA-Längenstandard als einzige Bande identifiziert werden.

### Beispiel 3: Vermehrung eines Teils einer doppelsträngigen DNA

Dieses Beispiel zeigt, daß die zu amplifizierende DNA Teil eines größeren DNA-Stückes sein kann, insbesondere eines Plasmids.

Als Template-DNA dient das Plasmid pUC19, das mit dem Restriktionsenzym Nar I (Boehringer Mannheim) linearisiert wurde. Als Oligonukleotidbausteine wurden M13rev (-48) (5'-dAgCggATAACAATTTCACACAggA-3') und M13iso (5'-dggCgTAATCATggTCATAgCTgTT-3') verwendet.

Es wurden 50 µl Reaktionsansätze in 0,5 ml Reaktionsgefäßen (Eppendorf, Safe-Lock) zusammenpipettiert, wobei die Reaktionsansätze die folgende Zusammensetzung aufwiesen.
10 mM (NH₄)₂SO₄
20 mM Tris-HCl (pH 8,8 bei 25 ° C)
2 mM MgSO₄
je 200 µM TTP, dGTP, dATP, dCTP
0,1% (M/V) Triton X-100
10 pmol Oligonukleotidbaustein M13rev (-48)
10 pmol Oligonukleotidbaustein M13iso
1 ng Template-DNA

Als Negativkontrolle wurde der gleiche Ansatz wie oben pipettiert, mit dem Unterschied, daß die Template DNA weggelassen wurde. Anschließend wurden die Reaktionsansätze mit 100 µl Mineralöl (SIGMA, M-3516) überschichtet. Die Ansätze wurden in einem Thermostatisiergerät (Gene Amp PCR System 9600, Perkin Elmer Cetus) 10 Minuten auf 95° C erwärmt, anschließend auf 70° abgekühlt wonach den Gemischen jeweils 2 Units des Enzyms Deep Vent_{R}(exo⁻) DNA Polymerase (New England Biolabs) zugesetzt wurden.

Danach wurden Proben und Negativkontrollen bei 70° °C 14 Stunden lang weiterinkubiert. 10 µl der Reaktionsgemische wurden auf einem 2%igen Agarosegel aufgetrennt und durch Färbung mit Ethidiumbromid und UV-Fluoreszenzanalyse sichtbar gemacht. Das Amplifizierungsprodukt konnte durch Vergleich mit einem DNA-Längenstandard als einzige Bande identifiziert werden.

### Beispiel 4: Nachweis eines RNA-Virus durch Vermehrung einer virusspezifischen cDNA-Sequenz

Dieses Beispiel zeigt, daß cDNA als Template für das erfindungsgemäße Verfahren verwendet werden kann. Es wird ein RNA-Virus (Grapevine Fanleaf Virus; GFLV) mittels spezifischer immobilisierter Antikörper aus einem Pflanzenrohextrakt isoliert, anschließend wird die virale RNA durch Hitze und Detergens freigelegt und mittels eines spezifischen Oligodeoxynukleotids revers transkribiert. Danach wird die cDNA isotherm amplifiziert.

### 4.1. Beschichten einer Oberfläche mit spezifischem Serum

120 µl des GFLV Serums (Bioreba, Basel, Schweiz; 1:500 in 0,1M Natrium-Carbonatpuffer, pH 9,6 verdünnt) wurden in Kunststoffreaktionsgefäß (MµltiTechnology, Salt Lake City, USA; 0,5 ml Volumen) pipettiert und 1 Stunde bei Raumtemperatur inkubiert. Danach wurden die Röhrchen dreimal mit TPBS (137 mM NaCl, 2,7 mM KC1, 10 mM Na-Phosphat. 0,1% M/V Tween 20; pH 7,4) und zweimal mit PBS (137 mM NaCl, 2,7 mM KCI, 10 mM Na-Phosphatpuffer, pH 7,4) gewaschen.

### 4.2. Inkubation der Proben in den vorbeschichteten Reaktionsgefäßen

Blattproben von Reben (Vitis vinifera cv. French Colombard) wurden mittels GFLV-ELISA (Bioreba, Basel) auf Infektion mit GFLV getestet.

In je ein vorbeschichtetes Röhrchen wurden je 100 µl Extrakt von Bättern einer infizierten und einer nicht infizierten Rebe pipettiert, es wurde 2 Stunden bei 4 ° C inkubiert, danach wurden die Antikörper dreimal mit TPBS und zweimal mit PBS gewaschen.

### 4.3. Verwendete Oligonukleotidbausteine

Die Oligonukleotidbausteine amplifizierten einen Teil des Hüllproteingens des Grapevine Fanleaf Virus. "3'-BINE" : 5'-dCTAgAgTgggAAACTggTTC-3' (EMBL Accession Nr. X16907, Basen 3543-3562) "GFLV5'-ENIB": 5'-dgTCCAggCTTTAgCTTTTATggTA-3' (EMBL Accession Nr. X16907, Basen 3519-3542)

### 4.4. Reverse Transkription

In die Reaktionsgefäße wurden 20 µl eines RT Puffers (50 mM Tris-HCl, pH 8,3, 7,5 mM MgCl₂) mit zusätzlich 1 mM dCTP, 1 mM dATP, 1 mM dGTP, 1 mM TTP, 20 pmol "3'-BINE" Oligonukleotidbaustein, 10 Units RNAase Inhibitor (Boehringer Mannheim, Deutschland), 10 Units des Enzyms AMV Reverse Transkriptase (Boehringer Mannheim, Deutschland) und 0,5% Triton X-100 pipettiert. Der Reaktionsansatz wurde 10 Minuten bei 65 °C inkubiert, anschließend 10 Minuten bei 42 ° C.

### 4.5.1. Isotherme DNA-Amplifizierung mit Deep Vent _{R} (exo⁻) DNA-Polymerase

In die jeweiligen Reaktionsansätze wurden 80 µl eines PCR Puffers [10 mM (NH₄)₂SO₄, 20 mM Tris-HCl, pH 8,8 bei 25 °C, 2 mM MgSO₄, 0,1% (M/V) Triton X-100] mit zusätzlich 20 pmol des Oligonukleotids "3'-BINE", 20 pmol des Oligonukleotids "GFLV 5'-ENIB" und 4 Units des Enzyms Deep Vent_{R}(exo⁻) DNA Polymerase (New England Biolabs) pipettiert. Die Reaktionsansätze wurden mit 70 µl leichtem Mineralöl (Sigma) überschichtet, sofort auf 68°C erwärmt und bei dieser Temperatur 16 Stunden inkubiert.

### 4.5.2. Isotherme DNA-Amplifizierung mit Tag DNA-Polymerase

In die jeweiligen Reaktionsansätze wurden 80 µl eines PCR Puffers (10 mM mM Tris-HCl, 50 mM KCl, 1,5 mM MgCl₂; pH 8,3 bei 20° C) mit zusätzlich 20 pmol des Oligonukleotids "3'-BINE", 20 pmol des Oligonukleotids "GFLV5'-ENIB" und 5 Units des Enzyms Taq DNA Polymerase (Boehringer Mannheim) pipettiert. Die Reaktionsansätze wurden mit 70 µl leichtem Mineralöl (Sigma) überschichtet, sofort auf 68° C erwärmt und bei dieser Temperatur 16 Stunden inkubiert.

### 4.6. Auswertung

10 µl jedes Reaktionsansatzes wurden auf einem 2%igem Agarosegel durch Elektrophorese aufgetrennt. Durch Ethidiumbromidfärbung und Fluoreszenzanregung UV-Licht wurden die Reaktionsprodukte sichtbar gemacht. Das Ergebnis war eine sichtbare Bande bei der Probe mit infiziertem Pflanzenmaterial, wobei die Länge der Bande 44 Basenpaare (durch Vergleich mit dem DNA-Standard "50 base-pair ladder", USB, Ohio) betrug. Die Proben mit Pflanzenextrakt aus nicht infizierten Pflanzen zeigten in der Elektrophorese keine Bande.

### Beispiel 5: Vermehrung eines Genabschnitts aus genomischer DNA

Dieses Beispiel zeigt, daß aus genomischer DNA ein Genabschnitt durch isotherme PCR amplifiziert werden kann.

### 5.1. Präparation der genomischen DNA

DNA der Zelllinie A-498 (ATCC HTB 44; humanes Nierenkarzinom) wurde nach Standardmethoden präpariert. Diese Präparation diente als Template-DNA.

### 5.2. Oligonukleotidbausteine

appr: 5'-dgCCTAATTCTCTCATAgTCTTAATTCCCAC-3'
appl-iso: 5'-dAggTAggTAAACTTgACTgCATgTTTCCAA-3'

### 5.3. Reaktion

Es wurde ein 100 µl Reaktionsansatz in einem 0,5 ml Reaktionsgefäß (Eppendorf, Safe-Lock) zusammenpipettiert wobei der Reaktionsansatz die folgende
Zusammensetzung aufwies.
10 mM (NH₄)₂SO₄
20 mM Tris-HCl (pH 8,8 bei 25 ° C)
2 mM MgSO₄
je 200 µM TTP, dGTP, dATP, dCTP
5 µM Biotin-16-dUTP (Boehringer Mannheim)
0,1% (M/V) Triton X-100
10 pmol Oligonukleotidbaustein appr
10 pmol Oligonukleotidbaustein appl-iso
5 µg Template-DNA

Als Negativkontrolle wurde der gleiche Ansatz wie oben pipettiert mit dem Unterschied, daß die Template-DNA weggelassen wurde. Anschließend wurde der Reaktionsansatz mit 100 µl Mineralöl (SIGMA, M-3516) überschichtet. Die Ansätze wurden in einem Thermostatisiergerät (Gene Amp PCR System 9600; Perkin Elmer Cetus) 10 Minuten auf 98° C erwärmt, anschließend auf 68°C abgekühlt, wonach den Gemischen jeweils 2 Units des Enzyms Deep Vent _{R}(exo⁻) DNA Polymerase (New England Biolabs) zugesetzt wurde. Danach wurden Probe und Negativkontrolle bei 68 ° C 14 Stunden lang weiterinkubiert.

### 5.4. Analyse der Reaktion

Je 90 µl der Reaktionsansätze wurden danach mittels einer Slot Blot Apparatur (Hoefer Scientific; PR600 Slot Blot) auf Nitrocellulose-Membran (Hoefer Scientific Instruments, TM-NC2) gespottet, die Membran danach 30 Minuten bei 80°C im Vakuumtrockenschrank inkubiert. Anschließend wurde die Membran in PBS mit 5%(M/V) Trockenmilchpulver (Fixmilch mager von Maresi, AT) 30 Minuten bei Raumtemperatur inkubiert und dreimal gewaschen. Nach einer weiteren Inkubation mit Streptavidin-Alkalische Phosphatase Konjugat (Amersham; 1:1000 verdünnt) in TPBS bei Raumtemperatur für 30 Minuten folgte fünfmaliges Waschen mit PBS und anschließender Färbereaktion mit Nitro-Blau-Tetrazolium (0,2 mg/ml) und 5-Brom-4-Chlor-3-Indoxyl- Phosphat (0,2 mg/ml), 4 mM MgCl₂ in 0,1 M Natriumcarbonatpuffer, pH 9,6. Nach 30 Minuten Färbezeit war bereits eine deutliche Blaufärbung an der Stelle des Reaktionsansatzes mit humaner genomischer DNA zu erkennen, während die Negativkontrolle keine Färbung zeigte.

Weitere Analysen des Reaktionsansatzes zeigten unspezifische Reaktionsprodukte, weshalb das Ausmaß der spezifischen Amplifizierung nicht genau abgeschätzt werden konnte.

Unter folgenden Reaktionsbedingungen kann die Amplifizierung aus genomischer DNA wesentlich spezifischer geführt werden (50µl Ansatz):
20 mM Tris.HCl pH 8,8
5 mM MgSO₄
dNTP's je 100 µM
0,1%(M/V) Triton X-100
10 pmol Oligonukleotidbaustein appr
10 pmol Oligonukleotidbaustein appl-iso
10 ng Template-DNA

20 Minuten Denaturierung bei 96°C, während dieser Zeit Zugabe von 2 Units Deep Vent _{R}(exo⁻) DNA Polymerase. Danach 1 Stunde Inkubation bei 77° C.

Das Produkt konnte als einzige Bande nach der Elektrophorese identifiziert werden.

### Beispiel 6: Nachweis einer Punktmutation

Dieses Beispiel zeigt, daß eine Punktmutation mittels des erfindungsgemäßen Verfahrens erfaßt werden kann.

### 6.1. Oligonukleotidbausteine:

"3'-BINE":	5'-dCTAgAgTgggAAACTggTTC-3'
"GFLV5'-ENIB":	5'-dgTCCAggCTTTAgCTTTTATggTA-3'
"GFLV5'-ENIBmut":	5'-dgTCCAggCTTTAgCTTTTATggTC-3'

### 6.2. Templates:

Als Templates dienten die synthetische Oligonukleotide. Die beiden Oligonukleotide unterscheiden sich in einer Base in Position 24.

BA: 5'-GTCCAGGCTTTAGCTTTTATGGTAGAACCAGTTTCCCACTCTAG-3'

IIIIIIIIIIIIIIIIIIIII IIIIIIIIIIIIIIIIIIII

BC: 5'-GTCCAGGCTTTAGCTTTTATGGTCGAACCAGTTTCCCACTCTAG-3'

### 6.3. Reaktionsansatz:

Es wurden 50 µl Reaktionsansätze in 0,5 ml Reaktionsgefäßen (Eppendorf, Safe-Lock) zusammenpipettiert, wobei die Reaktionsansätze folgende Zusammensetzung aufwiesen.
10 mM (NH₄)₂SO₄
20 mM Tris-HCl (pH 8,8 bei 25 ° C)
2 mM MgSO₄
je 200 µM TTP, dGTP, dATP, dCTP
0,1% (M/V) Triton X-100
10 pmol Oligonukleotidbaustein 3'BINE
10 pmol Oligonukleotidbaustein GFLV5'-ENIB oder
10 pmol Oligonukleotidbaustein GFLV5'-ENIBmut
ca. 1 fmol Template-DNA (BA oder BC)

Als Negativkontrolle wurde der gleiche Ansatz wie oben pipettiert, mit dem Unterschied, daß die Template-DNA weggelassen wurde. Anschließend wurden die Reaktionsansätze mit 100 µl Mineralöl (SIGMA, M-3516) überschichtet. Die Ansätze wurden in einem Thermostatisiergerät (Gene Amp PCR System 9600, Perkin Elmer Cetus) 10 Minuten auf 95° C erwärmt, anschließend auf 70°C abgekühlt und mit jeweils 2 Units des Enzyms Deep Vent_{R}(exo⁻) DNA Polymerase (New England Biolabs) versetzt. Danach wurden Proben und Negativkontrollen bei 68° C 14 Stunden lang weiterinkubiert. 10 µl der Reaktionsgemische wurden auf einem 2%igen Agarosegel aufgetrennt und durch Färbung mit Ethidiumbromid und UV-Fluoreszenzanalyse sichtbar gemacht. Das Amplifizierungsprodukt konnte durch Vergleich mit einem DNA-Längenstandard als einzige Bande identifiziert werden. Die Ergebnisse sind in Tabelle 2 zusammengefaßt.

**Tabelle 2:**

| Oligonukleotidkombination | Template | |
|---|---|---|
| | BA | BC |
| | | |
| 3'BINE/GFLVS'-ENIB | + | - |
| 3'BINE/GFLV5'-ENIBmut | - | + |
| + ...spezifische Bande an Elektrophorese-Gel | | |
| - ...keine Bande an Elektrophorese-Gel | | |

### Beispiel 7: Vermehrung einer linearen, einzelsträngigen DNA

Dieses Beispiel zeigt, daß es möglich ist, ein bestimmtes einzelsträngiges DNA-Molekül in vitro enzymatisch zu vermehren, auch wenn dessen Länge größer als die Summe der Oligonukleotidbausteine-Längen ist.

Dabei wird mit zwei Oligonukleotiden, die an zwei komplementäre DNA-Stränge binden, ein doppelsträngiges Produkt gebildet, ohne daß die Reaktionstemperatur verändert wird.

### 7.1. Reaktionsansatz:

Es wurden jeweils 50 µl Reaktionsansätze in 0,5 ml Reaktionsgefäßen (Eppendorf, Safe-Lock) zusammenpipettiert, wobei die Reaktionsansätze folgende Zusammensetzung aufwiesen.
50 mM KCl
10 m M Tris-H Cl (pH 8,3 bei 20 °C)
1,5 mM MgCl₂
je 200 µM TTP, dGTP, dATP, dCTP
10 pmol Oligonukleotidbaustein u67 (5'-dCAACCCAgTCAgCTCCTTCCggTgggCg-3')
10 pmol Oligonukleotidbaustein 174 (5'-dCgCTgCCggCACCTgTCCTACgAgTTgCATgA-3')
1 fmol Template-DNA:

5'CAACCCAgTCAgCTCCTTCCggTgggCgCgggCATgACTATCgTCgCCgCACT
TATgACTgTCTTCTTTATCATgCAACTCgTAggACAggTgCCggCAgCg-3'

Als Negativkontrollen dienten Reaktionsansätze, denen keine Template-DNA zugesetzt wurde. Die Oligonukleotidbausteine und das DNA-Template wurden von CODON genetic systems chemisch synthetisiert. Anschließend wurde der Reaktionsansatz mit 100 µl Mineralöl (Sigma, M-3516) überschichtet und auf dem Perkin Elmer Cetus Gene Amp PCR System 9600 Thermostatisiergerät auf 84° C erwärmt. 5 Units des Enzyms Taq DNA Polymerase (Perkin Elmer Cetus) wurden dem Gemisch nach Erreichen der Reaktionstemperatur zugesetzt. Anschließend wurden einzelne Proben und Negativkontrollen bei 84 ° C unterschiedlich lang weiterinkubiert, um einen Verlauf der Amplifizierung feststellen zu können. 10 µl der Reaktionsgemische wurden auf einem 2%igen Agarosegel aufgetrennt und durch Färbung mit Ethidiumbromid und UV-Fluoreszenzanalyse sichtbar gemacht. Das Amplifizierungsprodukt konnte durch Vergleich mit einem DNA-Längenstandard als einzige Bande identifiziert werden.

### Beispiel 8: Nachweis einer spezifischen DNA-Sequenz durch Template abhängige DNA-Ligation

### 8.1. Oligonukleotide und Templates:

Alle Oligonukleotid-Bausteine und Template-DNA wurden von CODON genetic systems bezogen. Die Oligonukleotide A und D sind am 5-Ende chemisch phosphoryliert.

Oligonukleotide:

A: 5'-pdTTgTgCCACgCggTTgggAATgTA-3'
B: 5'-dAgCAACgACTgTTTgCCCgCCAgTTg-3'
C: 5'-dTACATTCCCAACCgCgTggCACAAC-3'
D: 5'-pdAACTggCgggCAAACAgTCgTTgCT-3'

Template DNA: eine synthetische DNA mit folgender Sequenz wurde benutzt:

5'-dTACATTCCCAACCgCgTggCACAACAACTggCgggCAAACAgTCgTTgCT-
3'

Reaktionsansatz:
20 mM Tris-HCl (pH 7.5), 20 mM KCl, 10 mM MgCl₂ 1 mM DTT
0,1% NP-40 (detergent), 0,1 mM rATP
Oligonukleotide A, B, C, D: je 1 pmol
Template (100 fmol pro Reaktion)
Negativ-Kontrollen ohne Template-DNA

Die Reaktionsansätze (19µl) wurden auf 70° C aufgeheizt, danach wurde die Reaktion durch Zusatz von 4 units (1µl) *Pfu* DNA Ligase (Stratagene, USA) gestartet. Die Reaktion wurde 60 Minuten bei 70 °C durchgeführt. Nach der Inkubation wurden die Reaktionsansätze innerhalb einiger Minuten auf Raumtemperatur abkühlen gelassen. Danach wurden 10 µl jedes Ansatzes durch Gel-Elektrophorese analysiert (2% Standard EEO agarose in 0.5 fachem TBE Puffer bei 4°C and 5 V/cm, 10 min. Vorlauf).

### Resultat:

Die beschriebenen Ansätze des erfindungsgemäßen Verfahrens ergaben die gleichen Ergebnisse wie eine Ligase Chain Reaction mit 25 Zyklen (92 ° C-20 sec; 60° C-20 sec; Stratagene LCR Kit).

### Beispiel 9: Detektion von HIV I RNA mittels reverser Transkription und anschließender isothermer Amplifikation der cDNA

Nicht-infektiöse RNA wurde aus einem HIV I-Standardvektor *in vitro* transkribiert und in Verdünnungen für die Charakterisierung eines spezifischen RNA-Nachweises eingesetzt. Nach einer reversen Transkription der RNA zu cDNA führt die isotherme DNA Amplifikation zu DNA-Fragmentmengen, die mittels Agarosegelelektrophorese detektiert werden können. Die Nachweisgrenze von RNA liegt unter 0,001 attomol.

### 9.1. Herstellung der RNA durch In Vitro Transskription

Ausgangsmaterial zur *in vitro* Transkription stellt der Vektor *pBH10* dar, in dem eine knapp 9 kB lange cDNA von HIV I an der *Sacl* Stelle intergriert ist (Hahn et al, 1984, Nature 312:166 ). Der Vektor wurde über Standardtechniken amplifiziert und gereinigt, und mittels Restriktionsverdaues durch *BglI* linearisiert. Eine Transkription durch die SP6 RNA Polymerase beginnt auf dem derart linearisierten Vektor an der spezifischen Promotorregion, die 50 Basen vor der HIV - Sequenz liegt, und läuft bis zur nächsten stromabwärts gelegenen *Bgll* Stelle, die aufgeschnitten wurde. Das erhaltene Transkript umfaßt daher die gesamte, klonierte Virussequenz, plus 50 Basen stromaufwärts aus der multiplen Cloning site sowie 1470 Basen stromabwärts aus dem restlichen Vektor (*pBH10* ist ein Derivat von *pSP64*; Promega, USA). Die Stranglänge der so transkribierten RNA liegt daher bei ca. 10.200 Basen und das Molekulargewicht bei ca. 3.060.000 Dalton.

### 9.2. Reaktionsansatz

Zur *in vitro* Transkription wurden alle Lösungen und Kunststoffartikel von RNase - Aktivität durch Behandlung mit Diethylpyrocarbonat (DEPC) befreit. Eine Konzentration von 0,1 % (v/v) DEPC in Wasser oder der betreffenden wässrigen Lösung wirkte über Nacht bei Raumtemperatur ein, und nicht reagiertes DEPC wurde durch 120 Minuten Autoklavieren bei 121 °C zerstört.

Ein 100 µl Ansatz zur Transkription umfaßt folgende Komponenten:
0,5 µg linearisierter Vektor *pBH10/BgII*
je 1 mM ATP, CTP, GTP und UTP (Boehringer Mannheim, BRD)
40 Units RNAsin RNase Inhibitor (Boehringer Mannheim, BRD)
20 Units SP6 RNA Polymerase (Boehringer Mannheim, BRD)
11 µl 10-fach konzentrierter Reaktionspuffer (Boehringer Mannheim, BRD).

Dieser Ansatz wurde 14 Stunden bei 37°C inkubiert. Danach wurde 2 Stunden mit 50 Units DNase I bei 37°C weiter inkubiert. Durch Zugabe von 0,5 M EDTA Lösung pH 8,0 wurden 20 mM EDTA Endkonzentration eingestellt und der Ansatz für 10 Minuten auf 65°C erwärmt. Es folgten zwei Ethanolfällungen mit 0,1 vol 3 M Natriumacetat und 2,5 vol Ethanol. Nach der Bildung des Niederschlages bei -20 °C innerhalb von 30 Minuten wurde 30 Minuten bei 10.000 x g abzentrifugiert und das Pellet in 500 µl 70 % (v/v) Ethanol gespült. Nach der zweiten Fällung wurde in einem auf 25 µl reduzierten Volumen von Wasser wiederaufgelöst. Die Konzentration des Transkriptes wurde photometrisch bestimmt. Das für dieses Beispiel eingesetzte RNA-Prärarat zeigte nach Verdünnung auf das 100-fache Volumen eine Extinktionsdifferenz von 0,092 gegen Wasser bei 260 nm Wellenlänge. Dadurch kann auf eine RNA-Einzelstrangkonzentration von 0,4 µg/µl in der unverdünnten Lösung geschlossen werden. Die erhaltenen Präparate wurden in Aliquoten von 5 µl bei -20°C bis zu 2 Monate aufbewahrt.

### 9.3. Nachweis von RNA mit HIV I Sequenz über Isotherme Amplifikation

Der Nachweis umfaßt die Synthese von cDNA aus der viralen RNA und danach die isotherme Amplifikation der DNA. In diesem Beispiel startet die reverse Transkription mit dem Oligonukleotidbaustein HB101U699, und die Amplifikation läuft zwischen demselben Oligonukleotidbaustein und einem zweiten Oligonukleotidbaustein, HB101L725. Als Geräteausstattung sind Heizblöcke mit auf 0,5 °C genauer, konstanter Temperatur oder entsprechende Wasserbäder notwendig, sowie eine Standardausrüstung zur Agarosegelelektrophorese.

### 9.4. Reaktionsansatz

Das Transkript mit der Virussequenz wurde auf Eis mit Wasser so verdünnt, sodaß Lösungen mit 1 femtomol bis 0,001 attomol pro µl zur Verfügung standen. Die Verdünnungen wurden erst unmittelbar vor ihrer Verwendung hergestellt. Je 1 µl RNA-Verdünnung wurde in ein 0,5 ml Eppendorfgefäß überführt, in dem 7 µl reverser Transkriptionsmix vorlagen. Zusammen mit der Probe hatten die 8 µl Volumen für die reverse Transkription folgende Zusammensetzung:
10 mM Tris.HCl Puffer pH 8,8
50 mM KCl
4 mM MgCl₂
0,6 % (M/V) Triton X-100 (Sigma Chemicals, USA)
je 0,5 mM dATP, dCTP, dGTP und dTTP (Promega, USA)
8 pmol Oligonukleotidbaustein HB101U699
60 U RNAsin (Boehringer Mannheim, BRD)
2 U AMV Reverse Transkriptase (Boehringer Mannheim, BRD)

Die Reaktionsansätze wurden mit 50 µl leichtem Mineralöl (Sigma Chemicals, USA) überschichtet und auf einem Thermocycler (Trioblock TB1, Biometra, BRD) folgendem Programm unterworfen:

65°C für 10 Minuten, 42°C Hold. Nach 20 Minuten bei 42°C wurden die Eppendorfgefäße am Thermocycler belassen, und in zufälliger Reihenfolge mit 25 µl eines Amplifikationsmixes versetzt. Danach wurden die Reaktionsansätze 9 Stunden bei 72°C inkubiert.

Amplifikationsmix:
20 mM Tris.HCl Puffer pH 8,9
10 mM KCl
10 mM (NH₄)₂SO₄
4 mM MgSO₄
0.1 % (M/V) Triton X 100 (Sigma Chemicals, USA)
Je 0,2 mM dATP, dCTP, dGTP und dTTP (Boehringer Mannheim, BRD)
1 pmol/µl Oligonukleotidbaustein HB101L725
0,8 pmol/µl Oligonukleotidbaustein HB101U699
0,75 Units Taq DNA Polymerase pro 25 µl (Boehringer Mannheim, BRD)

Die verwendeten Oligonukleotidbausteine wurden auf einem ABI 392 DNA Sythesizer (Applied Biosystems, USA) mit folgender Sequenz hergestellt:

HB101U699 5'-dAgC CAT gCA AAT gTT AAA AgA gAC CA-3' und
HB101L725 5'-dCCC ATT CTg CAg CTT CTT CAT TgA-3'

Die amplifizierte DNA-Lösung wurde unter Standardbedingungen mittels Elektrophorese analysiert. Je 20 µl der Reaktionsansätze wurden mit 4 µl Probenpuffer versetzt und in einem 2 %-igem Agarosegel in 0,5-fachem TBE Puffer bei 5 V/cm aufgetrennt. Die DNA am Gel wurde durch Ethidiumbromidfärbung und UV-Fluoreszenzanalyse sichtbar gemacht.

| 0,5 x TBE Puffer: | Probenpuffer: |
|---|---|
| 45 mM Tris | 40 % (M/V) Saccharose |
| 45 mM Borsäure | 0,05 % (M/V) Bromphenolblau |
| 1 mM EDTA | |

Als Ergebnis zeigt Fig. 1 das Auftreten einer einzigen Bande von 50 Basen Länge in den Spuren 2 bis 8, die den RNA-Menge von 1 fmol (Spur 2) bis 0,001 amol (Spur 8) in Verdünnungsstufen von 1 fmol, 100 amol, 10 amol, 1 amol, 0,1 amol, 0,01 amol und 0,001 amol RNA mit HIV I Sequenz. Die Spuren 1 und 10 zeigen Referenzbanden von 50 und 83 Basen Länge aus DNA-Amplifikationen mit zyklischer, thermischer Denaturierung ("PCR"). Blindwerte wurden von 1 µl des Wassers gemacht, das zur Verdünnung eingesetzt wurde, und finden sich in Spur 9 und 11. Spur 12 zeigt das Resultat einer Blindamplifikation ohne das Enzym Reverse Transkriptase, und Spur 13 ist eine Blindprobe unter Weglassung der Taq DNA Polymerase.

### Beispiel 10: Nachweis von DNA mittels isothermer DNA Amplifikation auf einer Festphasen-Oberfläche

Das nachzuweisende DNA Fragment wurde isotherm zwischen zwei Oligonukleotiden amplifiziert, wobei eines der Oligonukleotide an einer Kunststoffoberfläche immobilisiert, das gegensinnige Oligonukleotid frei in Lösung vorlag. Zur Detektion wurde das immobilisierte Produkt mit einer markierten Sonde hybridisiert und die Markierung immunologisch nachgewiesen.

### 10.1. Isotherme Amplifikation von DNA auf Festphasen

Für die isotherme DNA Amplifikation wurde das Oligonukleotid HB101L725bio am 5'-Ende biotinyliert und an Mikrotiterplatten (Xenoprobe, Fa. Xenopore, USA) immobilisiert, die mit Streptavidin gecoated worden sind. Pro Näpfchen wurden 50 pmol Oligonukleotid in 100 µl PBSE.TW Puffer (siehe unten) gelöst und 1 Stunde langsam geschwenkt. Ungebundenes Oligonukleotid wurde durch achtmaliges Waschen mit 250 µl PBSE.TW entfernt.
1 x PBSE.TW, pH 7,4, enthält:
8 mM Na₂HPO₄
2 mM KH₂PO₄
137 mM NaCl
3 mM KCl
1,5 mM EDTA
0,1 % (M/V) Tween 20 (Merck, BRD)

Die gewaschenen Platten wurden für 30 Minuten bei 72°C in 200 µl/Näpfchen 1 x Amplifikationspuffer äquilibriert.
1 x Amplifikationspuffer:
20 mM Tris.HCl Puffer pH 8,9
10 mM KCl
10 mM (NH₄)₂SO₄
2 mM MgSO₄
0.1 % (M/V) Triton X-100 (Sigma Chemicals, USA)

Für die Amplifikationsreaktion wurde dieser Äquilibrierungspuffer durch 100µl/Näpfchen Amplifikationsmix ersetzt, und das nachzuweisende Fragment in drei Verdünnungen als jeweils 1 µl Lösung zugegeben. Die Mikrotiterplatte wurde kurz geschwenkt, mit Klebefolie (Nunc, Dänemark) gegen Verdunstung verschlossen und für 15 Stunden bei 72°C inkubiert.

Amplifikationsmix:
1 x Amplifikationspuffer mit
je 0,2 mM dATP, dCTP, dGTP und dTTP (Boehringer Mannheim, BRD)
1 pmol/µl Oligonukleotidbaustein HB101U699
1,5 Units Taq DNA Polymerase pro 100 µl (Boehringer Mannheim, BRD)

Die verwendeten Oligonukleotidbausteine wurden auf einem ABI 392 DNA Sythesizer (Applied Biosystems, USA) mit folgender Sequenz hergestellt: HB101U699: 5'-dAgC CAT gCA AAT gTT AAA AgA gAC CA-3'; HB101L725bio: 5'-dCCC ATT CTg CAg CTT CTT CAT TgA-3', mit Biotinylierung am 5'-Ende; Detektionsoligo: 5'-dgTT AAA AgA gAC CAT CAA TgA ggA Ag-3', mit 6-Carboxyfluoresceinmarkierung am 5'-Ende.

### 10.2. Nachweis der immobilisierten, amplifizierten DNA auf Festphasen

Die Mikrotiterplatten wurden nach der Amplifikation dreimal mit PBSE.TW gewaschen, und im gleichen Puffersystem für eine Hybridisierung vorkonditioniert. Dazu wurde 0,1 mg/ml frisch denaturierte Heringssperma DNA (Boehringer Mannheim) in PBSE.TW gelöst und pro Näpfchen 200 µl Konditionierungslösung für 30 Minuten bei 50°C unter Schwenken mit 500 Upm inkubiert. Zur Hybridisierung wurden pro Näpfchen 100 µl Konditionierungslösung eingesetzt, in denen 5 pmol Detektionsoligo vorlagen. Nach 30 Minuten Hybridisierung unter oben beschriebenen Bedingungen wurden die Platten fünfmal mit 250 µl PBSE.TW für 10 Minuten bei 50°C gewaschen.

Für den immunologischen Nachweis wurden die Platten mit 250 µl 3% Casiton (Difco, USA) in PBSE (Blockinglösung) für 30 Minuten bei Raumtemperatur geblockt, und danach mit Anti-Fluoresceinisothiocyanat-Antikörper, gekoppelt an Meerrettich Peroxidase (Dako, Dänemark), der 1 : 2000 in Blocking Lösung verdünnt worden war, inkubiert.

Die Platten wurden fünfmal mit PBSE.TW gewaschen, und schließlich mit 110 µl Peroxidase Substrat (ImmunoPure, Pierce, USA) 25 Minuten inkubiert. Nach Abstoppen der Enzymreaktion mit 110 µl 4N Schwefelsäure wurden die Extinktionsdifferenzen bei 450 nm gemessen.

Abzüglich der Extinktion, die vom Antikörperkonjugat nach einer Hybridisierungsprozedur ohne vorangegangene DNA Amplifikation verursacht wird, ergaben sich folgende Werte:

| Eingesetzte Templatmenge | Prozedur | Extinktionsdifferenz |
|---|---|---|
| 1 1 fmol | komplett | 0,446 |
| 2 100 amol | komplett | 0,318 |
| 3 10 amol | komplett | 0,112 |
| 4 0 amol | komplett | 0,081 |
| 5 1 fmol | ohne Taq DNA Polymerase | 0.076 |
| 6 1 fmol | ohne Taq DNA Polymerase | |
| | ohne HB101U699 | 0,048 |
| 7 0 amol | ohne Amplifikationsreaktion | 0,000 |

### 10.3. Interpretation der Daten

In Korrelation zu der angebotenen Templatmenge wurden ansteigende Extinktionsdifferenzen gemessen (vgl. Zeilen 1,2,3,4). Das erfindungsgemäße Verfahren ist in der Lage, eine isotherme Amplifizierung geringer Templatemengen an einer festen Oberfläche durchzuführen. Um die Signifikanz der Meßwerte zu demonstrieren, wurden zusätzliche Blindwerte mitgeführt. Die inkompletten Blindwerte unter Weglassung der Taq DNA Polymerase (Zeile 5) bzw. ohne Taq DNA Polymerase und ohne Oligonukleotid HB101U699 (Zeile 6) basieren auf dem Einsatz der Höchstmenge an Template (1 fmol), und liegen niedriger als der Blindwert einer kompletten Reaktion ohne Template (Zeile 4). Die erhaltenen Meßwerte (Zeile 1,2,3,4) werden daher nicht in störendem Ausmaß von Nukleotidkomponenten aus der Amplifikation und der Hybrbridisierung überlagert.

### Beispiel 11: Nachweis von DNA mittels Isothermer Amplifikation mit einem teilweise überlappendem Paar von Oligonukleotiden

Dieses Beispiel demonstriert die Einsatzfähigkeit der erfindungsgemäßen isothermen DNA Amplifikation bei Verwendung einer Oligonukleotidkombination, deren Basenabfolge teilweise redundant ist. Durch die Überschneidung der zu den Oligonukleotiden homologen Regionen auf der Target-DNA wird ein Fragment amplifiziert, dessen Länge unter der Summe der Längen der beiden Oligonukleotide liegt.

### 11.1. Oligonukleotidbausteine:

Die Olignukleotide wurden auf einem ABI 392 DNA Sythesizer (Applied Biosystems, USA) mit folgender Sequenz sythetisiert :

HIVLAP1 5'-dAAT AgT AAg AAT gTA TAg CCC TAC CAg CAT-3' und
HIVLAP2 5'-dTTT Tgg TCC TTg TCT TAT gTC CAg AAT-3'

Die Oligonukleotidbausteine wurden so ausgewählt, daß für die Amplifikation die zwei jeweils 3'-terminalen Basen (unterstrichen) eine redundante Information darstellen. Das Amplifikationsprodukt hat daher auf dem vom Oligonukleotidbaustein HIVLAP1 angeführten Strang folgende Sequenz:

5'-dAAT AgT AAg AAT gTA TAg CCC TAC CAg CAT TCT ggA CAT AAg
ACA Agg ACC AAA A-3'

Als Template für die isotherme Amplifikation wurde ein doppelsträngiges DNA Fragment eingesetzt, welches mit ebendiesen Oligonukleotiden aus HIV I - cDNA mittels zyklischer, hitzedenaturierender Amplifikation ("PCR") hergestellt worden war. Als Template für diese Reaktion diente das Plasmid *pBH10* (Hahn et al. 1984, Nature 312:166).

Je 1 µl der Verdünnungen dieses Fragmentes wurden mit je 33 µl Amplifikationsmix (siehe unten) gemischt, mit 50 µl Mineralöl leicht (Sigma Chemicals, USA) überschichtet und 15 Stunden bei 70°C inkubiert.

Amplifikationsmix :
20 mM Tris.HCl Puffer, pH 8,9
10 mM KCl
10 mM (NH₄)₂SO₄
2 mM MgSO₄
0.1 % (M/V) Triton X-100 (Sigma Chemicals, USA)
Je 0,2 mM dATP, dCTP, dGTP und dTTP (Boehringer Mannheim, BRD)
0,2 pmol/µl Oligonukleotidbaustein HIVLAP1
0,2 pmol/pl Oligonukleotidbaustein HIVLAP2
0,5 Units Taq DNA Polymerase pro 33 µl (Boehringer Mannheim, BRD)

Die amplifizierte DNA-Lösung wurde unter Standardbedingungen mittels Elektrophorese analysiert. Je 20 µl der Reaktionsansätze wurden mit 4 µl Probenpuffer versetzt und in einem 2%-igem Agarosegel in 0,5-fachem TBE Puffer bei 5 V/cm aufgetrennt. Die DNA am Gel wurde durch Ethidiumbromidfärbung und

UV-Fluoreszenzanalyse sichtbar gemacht.

| 0,5 x TBE Puffer: | Probenpuffer: |
|---|---|
| 45 mM Tris | 40 % (M/V) Saccharose |
| 45 mM Borsäure | 0,05 % (M/V) Bromphenolblau |
| 1 mM EDTA | |

Die Elektrophorese zeigte bei einer Templatmenge von 10 femtomol eine deutliche Bande amplifizierter DNA, bei einer Templatmenge von 1 femtomol eine gut detektierbare Produktbande, und beim Einsatz von Wasser als Blindwert keine Bande. Die korrekte Länge der gebildeten Produkte (55 Basen) wurde durch die leicht geringere Wanderungsgeschwindigkeit im Vergleich zu einem 50 Basen langem Standard abgeschätzt.

### Beispiel 12: Detektion von HIV I RNA durch reverse Transkription und anschließender isothermer Amnlifikation der cDNA mit modifizierten Oligonukleotiden

Nicht-infektiöse RNA wurde aus einem HIV I - Standardvektor *in vitro* transkribiert und für die Charakterisierung eines spezifischen RNA-Nachweises eingesetzt. Nach einer reversen Transkription der RNA zu cDNA führt die isotherme DNA Amplifikation zu biotinylierten DNA-Fragmenten. Die gebildeten Fragmente wurden an Streptavidinbeladenen Mikrotiterplatten gebunden, mit einer Fluorescein-markierten Sonde hybridisiert, und über ein Anti-Fluorescein-Antikörper-Enzym-Konjugat nachgewiesen. Die Nachweisgrenze von RNA liegt unter 0,1 attomol.

### 12.1. Herstellung der RNA durch in vitro Transkription

Dieser Schritt ist im Beispiel 9, Punkt 9.1 und 9.2, beschrieben und wurde ohne Modifikationen für dieses Beispiel übernommen.

### 12.2. Isotherme Amplifikation von Nukleinsäuren aus RNA mit HIV I Sequenz

Dieser Schritt ist im Beispiel 9, Punkt 9.3 und 9.4, beschrieben und wurde mit zwei Änderungen übernommen:
12.2.1) Der zweite Oligonukleotidbaustein zum Ablauf der isothermen Amplifikation ist am 5'-Ende biotinyliert: BH101L725Bio
12.2.2) Zwei Verdünnungen des Transkriptes mit der Virussequenz wurden hergestellt, und in der Prozedur verwendet: 10 attomol pro µl und 0,1 attomol pro µl.

Die verwendeten Oligonukleotidbausteine wurden auf einem ABI 392 DNA Sythesizer (Applied Biosystems, USA) mit folgender Sequenz hergestellt: HB101U699: 5'-dAgC CAT gCA AAT gTT AAA AgA gAC CA-3' und HB101L725Bio: 5'-dCCC ATT CTg CAg CTT CTT CAT TgA-3', Biotinylierung am 5'-Ende. Detektionssonde: 5'-dgTT AAA AgA gAC CAT CAA TgA ggA Ag-3', mit 6-Carboxyfluoresceinmarkierung am 5'-Ende (Synthese mit 6-FAM Amidite, Applied Biosystems, USA).

### 12.3. Nachweis der amplifizierten DNA auf Festphasen

Zur Bindung des biotinylierten DNA Produktes wurden mit Streptavidin beladene Mikrotiterplatten (Xenoprobe, Fa. Xenopore, USA) zwei mal mit PBSE.TW gewaschen. Die amplifizierten DNA-Lösungen wurden mit 9 Volumina PBSE.TW Puffer verdünnt, und 1 µl dieser Verdünnung in 100 µl PBSE.TW durch 5 Minuten langes Kochen denaturiert. Pro Näpfchen der Mikrotiterplatte wurden die kompletten 100 µl denaturierte Lösung einpipettiert und bei 500 rpm 30 min geschwenkt. Die Platten wurden zwei mal mit PBSE.TW gewaschen und der Hybridisierung mit dem Detektionsoligo unterzogen.

Das weitere Nachweisverfahren der DNA Fragmente über Hybridisierung und Antikörperreaktion ist im Beispiel 10 Punkt 10.2 beschrieben und wurde mit folgenden drei Änderungen durchgeführt:
12.3.1 Die Konzentration der Detektionssonde betrug 2,5 picomol pro Näpfchen.
12.3.2 Die Verdünnung des Anti-Fluoresceinisothiocyanat-Antikörpers, gekoppelt an Meerrettich Peroxidase, betrug 1 zu 5000.
12.3.3 Die Inkubationszeit zur Farbentwicklung im Peroxidase Substrat belief sich auf 80 Minuten.

### 12.4. Interpretation der Daten

In Korrelation zu der angebotenen Templatemenge werden ansteigende Extinktionsdifferenzen gemessen (Zeilen 1,2,3). Das hier beschriebene Verfahren ist in der Lage, eine isothenne Amplifikation von geringen Nukleinsäuremengen durchzuführen, bei der ein durch Biotinylierung modifizerter Oligonukleotidbaustein in das Produkt inkorporiert wird. Damit ist die amplifizierte DNA jeder Nachweisreaktion zugänglich, bei der der Einsatz modifizierter Oligonukleotidbausteine Voraussetzung ist.

Bei diesem Beispiel wird die amplifizierte DNA über den Biotin-Rest an einer Festphase gebunden und mittels Hybridisierung quantifiziert.

| | Eingesetzte Templatemenge | Amplifikations-Prozedur | Extinktions-differenz |
|---|---|---|---|
| 1 | 10 amol | komplett | 1,516 |
| 2 | 0,1 amol | komplett | 0,376 |
| 3 | 0 amol | komplett | 0,180 |
| 4 | 0 amol | keine Taq DNA Polymerase | 0.105 |
| 5 | Leerwert mit Konjugat | | 0,087 |
| 6 | Leerwert ohne Konjugat | | 0,012 |

Die Blind- und Leerwerte erklären sich wie folgt:

Zeile 3 stellt den komplettesten Blindwert dar, bei dem nur das RNA Template weggelassen wurde. Zeile 4 entspricht Zeile 3 unter weiterer Weglassung der Taq DNA Polymerase, wobei in Relation zu den Werten von Zeile 1,2 und 3 das Zehnfache Volumen der Amplifikationsreaktion Verwendung fand. Zeilen 5 und 6 charakterisieren die Blindwerte aus dem Hybridisierungsnachweis mit und ohne dem Antikörper-Peroxidase-Konjugat.

### Beispiel 13: Sequenzspezifische DNA Detektion durch isotherme Amplifikation mit modifizierten Oligonukleotiden

Target DNA wurde unter Verwendung von biotinylierten Oligonukleotiden isotherm amplifizert und die gebildeten Fragmente an Oligonukleotid-beladenen Mikrotiterplatten mittels Hybridisierung gebunden. Der Nachweis der amplifizierten DNA erfolgte über ein Streptavidin-Enzym-Konjugat, welches an dem modifizierten Oligonukleotidbaustein bindet.

### 13.1. Isotherme Amplifikation von DNA mit modifizierten Oligonukleotiden

Als Template für die isotherme Amplifikation dienten DNA-Fragmente, die unter Einsatz der Oligonukleotidbausteine Appl30 und Appr30 aus humaner DNA mittels hitzedenaturierender Amplifikation ("PCR") gewonnen worden sind. Templateverdünnungen mit 20, 2 und 0,2 femtomol pro µl wurden hergestellt und jeweils 1 µl mit 50 µl Amplifikationsmix versetzt. Die Mischungen wurden mit 50 µl Mineralöl (Sigma Chemicals, USA) überschichtet und 90 Minuten bei 77 °C in einem Thermocycler (TB1, Biometra, BRD) inkubiert.

Amplifikationsmix:
20 mM Tris.HCl Puffer pH 8,8
2 mM MgSO₄
0.1 % (M/V) Triton X-100 (Sigma Chemicals, USA)
je 0,2 mM dATP, dCTP, dGTP und dTTP (Promega, USA)
0,2 pmol/µl Oligonukleotidbaustein Appl30Bio
0,2 pmol/µl Oligonukleotidbaustein Appr30
2 Units Deep Vent_{R}(exo⁻) DNA Polymerase pro 50 µl (NEB, USA)

Die verwendeten Oligonukleotidbausteine wurden auf einem ABI 392 DNA Sythesizer (Applied Biosystems, USA) mit folgender Sequenz hergestellt: Appl30Bio: 5'-dAgg TAg gTA AAC TTg ACT gCA TgT TTC CAA-3'; Am 5'-Ende biotinyliert, Appl30: 5'-dAgg TAg gTA AAC TTg ACT gCA TgT TTC CAA-3', Appr30: 5'-dgCC TAA TTC TCT CAT AgT CTT AAT TCC CAC-3', und AppCap: 5'-dgTC TTA ATT CCC ACT Tgg AAA CAT gCA-3', am 5'-Ende chemisch phosphoryliert.

### 13.2. Nachweis der amplifizierten DNA auf Festphase

Die amplifizierte DNA wurde mittels Hybridisierung an der Oberfläche von Mikrotiterplatten gebunden. Dazu erfolgte eine chemische Kopplung eines "Einfang"-Oligonukleotides (AppCap, siehe oben) an die Platten.

KOPPLUNGPROZEDUR: Mikrotiterplatten mit sekundären Aminogruppen (CovaLink NH, Nunc, Dänemark) wurden mit 5 pmol Oligonukleotidbaustein AppCap pro Well in 50 µl Imidazolpuffer (0,1 M N-Methyl-Imidazol, pH 7,0) beschickt. Die Kopplung wurde durch Zugabe von 50 µl einer 2%-igen (M/V) 1-Ethyl-3-(3-dimethylaminopropyl)-carbodiimid Lösung in Imidazolpuffer gestartet und 3 Stunden bei Raumtemperatur geschüttelt. Die Reinigung der Platten erfolgte mittels sechsmaligem Waschen mit 0,4 N NaOH plus 0,25 % (M/V) Natriumdodecylsulfat, sechsmaligem Waschen mit PBSE.TW Puffer, und dreimaligem Waschen mit destilliertem Wasser.
1 x PBSE.TW, pH 7,4, enthält:
8 mM Na₂HPO₄
2 mM KH₂HPO₄
137 mM NaCl
3 mM KCl
1,5 mM EDTA
0,1 % (M/V) Tween 20 (Merck, BRD)

NACHWEISREAKTION Zur Vorkonditionierung der Platte für die Hybridisierung wurden 0,1 mg/ml frisch denaturierte Heringssperma DNA (Boehringer Mannheim) in PBSE.TW gelöst und pro Näpfchen 170 µl dieser Konditionierungslösung für 15 Minuten bei 50°C unter Schwenken mit 500 RPM inkubiert.

Für die Hybridisierung wurden die amplifizierten DNA-Lösungen durch 5 Minuten langes Kochen denaturiert und pro Näpfchen 1 µl denaturierte Lösung in 100 µl frischer Konditionierungslösung eingesetzt. Nach 120 Minuten Hybridisierung bei 50°C und Schwenken mit 500 RPM wurden die Platten dreimal mit 200 µl PBSE.TW für 10 Minuten bei 50°C gewaschen.

Für den Nachweis des nunmehr immobilisierten, biotinylierten Stranges des isothermen Amplifikationsproduktes wurden die Platten mit 150 µl 1,5 % Casiton (Difco, USA) in PBSE.TW (Blocking Lösung) für 10 Minuten bei Raumtemperatur geblockt, und danach mit einem Streptavidin-Alkalische Phosphatase-Konjugat (Amersham, UK) für 30 Minuten inkubiert, das 1 : 2000 in Blocking Lösung verdünnt worden war.

Die Platten wurden sechsmal mit PBSE.TW gewaschen, und in 100 µl Färbelösung 90 Minuten lang entwickelt. Die Extinktionsdifferenzen wurden photometrisch bei 405 nm bestimmt.

Färbelösung:
50 mM Tris.NaOH, pH 10
150 mM NaCl
2 mM MgSO₄
7 mM 4-Nitrophenylphosphat

### Ergebnis:

| | Eingesetzte Templatmenge | Amplifikations-Prozedur | Extinktions-differenz |
|---|---|---|---|
| 1 | 20 fmol | komplett | 0,573 |
| 2 | 2 fmol | komplett | 0,232 |
| 3 | 0,2 fmol | komplett | 0,055 |
| 4 | 0 fmol | ohne Deep Vent_{R}(exo⁻)Pol. | 0,030 |
| 5 | 0 fmol | Hybridisierungsleerwert mit Konjugat | 0,019 |

### 13.3. Interpretation der Daten

In Korrelation zu der angebotenen Templatemenge werden ansteigende Extinktionsdifferenzen gemessen (Zeilen 1,2,3). Das hier beschriebene erfindungsgemäße Verfahren ist in der Lage, eine isotherme Amplifikation von geringen Nukleinsäuremengen durchzuführen, bei der ein durch Biotinylierung modifizerter Oligonukleotidbausteine in das Produkt inkorporiert wird. Damit ist die amplifizierte DNA jeder Nachweisreaktion zugänglich, bei der der Einsatz modifizierter Oligonukleotidbausteine Voraussetzung ist. Bei diesem Beispiel bindet ein mit Streptavidin konjugiertes Markerenzym (Alkalische Phosphatase) an der Biotinylierung der amplifizierten DNA. Die Spezifität des Nachweises der amplifizierten DNA ist über die Blindwerte 4 und 5 sichergestellt, wo eine Blindamplifikation ohne Template und ohne Deep Vent_{R}(exo⁻) DNA Polymerase (Zeile 4) bzw. eine Blindhybridisierung ohne Zusatz von Amplifikationsmix (Zeile 5) deutlich niedrigere Extinktionsdifferenzen ergeben, als der niedrigste Meßwert (Zeile 3).

### Beispiel 14:Amplifikationsrate des erfindungsgemäßen Verfahrens innerhalb einer Stunde

Dieses Beispiel zeigt die hohe Amplifikationsrate, die innerhalb kurzer Reaktionszeiten möglich ist.

Es wurden zwei unabhängige Oligonukleotid/Template Kombinationen gewählt:

### Kombination 1:

Oligonukleotidbausteine:
"APPR30":5'-dAgg TAg gTA AAC TTg ACT gCA TgT TTC CAA-3'
"APPL30":5'-dgCC TAA TTC TCT CAT AgT CTT AAT TCC CAC-3'

und Template "APP": ein synthetisches, doppelsträngiges DNA-Stück mit folgender Sequenz wurde verwendet:

5'-dAgg TAg gTA AAC TTg ACT gCA TgT TTC CAA gTg ggA ATT AAg ACT
ATg AgA gAA TTA ggC-3'

### Kombination 2:

Oligonukleotidbausteine:
"synth1": 5'-dTAg gTT ACT TAg CAT TCT CCA CTT AgA CgA-3

"synth2": 5'-dCTA gAg ATA TTg TTC CAT ACA gAT CCC AgT-3

und Template "Synth",ein synthetisches, doppelsträngiges DNA-Stück mit folgender Sequenz wurde verwendet

5'-dTAg gTT ACT TAg CAT TCT CCA CTT AgA CgA ACT ggg ATC TgT ATg
gAA CAA TAT CTC TAg-3

Reaktionsansätze (50µl):
20 mM Tris.HCl pH 8,8
5 mM MgSO₄
dNTP's je 100 µM
0,1%(M/V) Triton X-100
Oligonukleotidbausteine je 0,2 µM

Template, passend zu den entsprechenden Oligonukleotidbausteinen: in verschiedenen Ansätzen wurden verschiedene Templatemengen zugesetzt: 200 amol, 20 amol, 2 amol

Als Negativkontrolle diente ein Reaktionsansatz, dem keine Template-DNA zugesetzt wurde.

### 14.1. Durchführung:

Die Reaktionsansätze wurden auf 77°C erhitzt, mit Mineralöl/leicht überschichtet und durch Zugabe von je 2 units DeepVent_{R}(exo⁻) DNA-Polymerase (NEB, USA) gestartet.

Nach einer Stunde auf 77 °C wurden die Reaktionsgefäße innerhalb einiger Minuten auf Raumtemperatur gekühlt und 10 µl davon gelelektrophoretisch analysiert (% Standard EEO-Agarose in 0,5fachem Tris-Borat-EDTA (TBE) Puffer bei 4 °C und 5 V/cm, 10 min Vorlauf). Bei allen Reaktionsansätzen mit zugegebenem passendem Template ist eine deutliche Bande bei 60 bp zu sehen, die Negativkontrolle zeigt keine Bande.

### Beispiel 15: Vermehrung verschieden langer linearer, doppelsträngifer DNA

Dieses Beispiel zeigt, daß es möglich ist, mit einem bestimmten Oligonukleotidpaar unterschiedlich lange Templatesequenzen zu amplifizieren.

### Reaktionsansätze:

Es wurden jeweils 50 µl Reaktionsansätze in 0,5 ml Reaktionsgefäßen (Eppendorf, Safe-Lock) zusammenpipettiert. Die Reaktionsansätze wiesen dabei folgende Zusammensetzung auf:
20 mM Tris-HCl (pH 8,8 bei 25 °C)
2 mM MgSO₄
je 250 µM TTP, d GTP, d ATP, d CTP
0,1% (M/V) Triton X-100
10 pmol Oligonukleotidbaustein u676: 5'-dgAgCCTTCAACCCAgTCAgCTCCTTCCggTgggCgCggggC-3'
10 pmol Oligonukleotidbaustein 1755 5'-dCgCCgAAAATgACCCAgAgCgCTgCCggCACCTgTCCTACgAgTTgCATg-3'
20 fmol Template-DNA

Als Template DNAs wurden in den verschiedenen Ansätzen folgende doppelsträngige synthetische DNA benutzt:
"iso2", "iso2+1", "iso2+5" (Sequenzen siehe Anhang)

Als Negativkontrollen dienten Reaktionsansätze, denen keine Template-DNA zugesetzt wurde.

Anschließend wurden die Reaktionsansätze mit je 100 µl Mineralöl (SIGMA, M-3516) überschichtet und auf einem Perkin Elmer Cetus Gene Amp PCR System 9600 Thermostatisiergerät auf 84 °C erwärmt. Nach Erreichen der Reaktionstemperatur wurden dem Gemisch 2 Units des Enzyms Deep Vent_{R}(exo⁻) DNA Polymerase (New England Biolabs) zugesetzt. Anschließend wurden Proben und Negativkontrollen bei 84°C 14 Stunden lang weiterinkubiert.

### Visualisierung der Ergebnisse:

Die Ansätze wurden unter Standardbedingungen mittels Elektrophorese analysiert. Je 20 µl der Reaktionsansätze wurden mit 4 µl Probenpuffer versetzt und in einem 2 %-igem Agarosegel in 0,5-fachem TBE Puffer bei 5 V/cm aufgetrennt. Die DNA am Gel wurde durch Ethidiumbromidfärbung und UV-Fluoreszenzanalyse sichtbar gemacht.

0,5 x TBE Puffer (45 mM Tris; 45 mM Borsäure;1 mM EDTA)

Probenpuffer: 40 % (M/V) Saccharose; 0,05 % (M/V) Bromphenolblau

### Ergebnisse:

Die Elektrophorese zeigte bei den Proben mit Template "iso2" eine deutliche Bande, bei den Proben mit Template "iso2+1" eine schwächere Bande, bei den Proben mit Template "iso2+5" eine sehr schwache Bande in der dem Produkt entsprechenden Höhe (die korrekte Länge der gebildeten Produkte wurde durch Vergleich zu einer Standard DNA; 50 base-pair ladder von United States Biochemicals, Ohio, USA, abgeschätzt). Die Reaktionsansätze ohne Template war keine Bande zu sehen.

### Beispiel 16: Detektion von HIV I Sequenzen durch isotherme Amnlifikation mit modifizierten Oligonukleotidbausteinen

Dieses Beispiel zeigt die Amplifizierung spezifischer Sequenzen mit markierten Oligonukleotidbausteinen. Die beiden spezifischen Oligonukleotidbausteine sind unterschiedlich markiert, sodaß das Amplifizierungsprodukt zwei verschiedene Markierungen besitzt. Eine dieser Markierung (Biotin) wird verwendet, um das Produkt einfach an eine Festphase zu immobilisieren, sodaß es einer weiteren Detektion über die zweite Markierung (Fluorescein) zugänglich ist. Über eine Verdünnungsreihe von spezifischen Targetmolekülen wird gezeigt, daß die Menge an Produkt in einem bestimmten Bereich proportional der Ausgangsmenge Template ist.

Die verwendeten Oligonukleotidbausteine wurden auf einem ABI 392 DNA Sythesizer (Applied Biosystems, USA) mit folgender Sequenz hergestellt:

HB101U699FAM: Fluorescein-5'-dAgC CAT gCA AAT gTT AAA AgA gAC CA-
3' und
HB101L725Bio: Biotin-5'-dCCC ATT CTg CAg CTT CTT CAT TgA-3'

Das verwendete Template war doppelsträngige, synthetische DNA mit folgender Sequenz:

5'-dAgCCATgCAAATgTTAAAAgAgAC CATCAATgAAgAAgCTgCAgAATggg-3'

### Rektionsansatz:

Es wurden 50 µl Reaktionsansätze in 0,5 ml Reaktionsgefäßen (Eppendorf, Safe-Lock) zusammenpipettiert, wobei die Reaktionsansätze die folgende Zusammensetzung aufwiesen.
20 mM Tris-HCl (pH 8,8 bei 25 ° C)
5 mM MgSO₄
je 200 µM TTP, dGTP, dATP, dCTP
0,1% (M/V) Triton X-100
10 pmol Oligonukleotidbaustein HB101U699FAM
10 pmol Oligonukleotidbaustein HB101L725Bio
Template-DNA

Als Negativkontrolle wurde der gleiche Ansatz wie oben pipettiert, mit dem Unterschied, daß die Template DNA weggelassen wurde. Anschließend wurden die Reaktionsansätze mit 100 µl Mineralöl (SIGMA, M-3516) überschichtet. Die Ansätze wurden in einem Thermostatisiergerät (Gene Amp PCR System 9600, Perkin Elmer Cetus) auf 74° C erwärmt, anschließend jeweils 2 Units des Enzyms DeepVent_{R}(exo⁻) DNA Polymerase (New England Biolabs) zugesetzt und 90 Minuten inkubiert.

10 µl der Reaktionsgemische wurden auf einem 2%igen Agarosegel aufgetrennt und durch Färbung mit Ethidiumbromid und UV-Fluoreszenzanalyse sichtbar gemacht. Das Amplifizierungsprodukt konnte durch Vergleich mit einem DNA-Längenstandard als einzige Bande identifiziert werden.

### Nachweis durch Festphasen-Test in Microtiterplatten:

Streptavidin-beschichtete Mikrotiterplatten (Xenoprobe, Fa. Xenopore, USA) wurden mit PBS gewaschen, danach wurden 100 µl der einzelnen Proben der Amplifizierungsreaktionen, die in TPBS jeweils 1:10, 1:40, bzw. 1:4000 verdünnt worden waren, in die Näpfchen pipettiert und 25 min. bei Raumtemperatur geschüttelt. Anschließend wurde die Platte dreimal mit TPBS gewaschen. In die Näpfchen wurde danach je 100 µl Konjugatverdünnung pipettiert (anti-Fluorescein-Alkalische Phosphatase, Boehringer Mannheim, Deutschland; 1: 10000 in TPBS). Nach einer Stunde Inkubation unter Schütteln wurde die Platte wieder dreimal mit TPBS gewaschen. Anschließend wurde einmal mit PBS gewaschen. Danach wurde in die Näpfchen jeweils 100 µl Färbelösung (LumiPhos plus von Lumigen, USA) pipettiert.

Nach einer halben Stunde Inkubation wurde die Lumineszenz auf einem Luminometer (Lucy1 von Anthos, AT) gemessen. Zur Ermittlung des Rauschens wurden die Negativansätze verwendet.

### Ergebnis:

| Anzahl der Moleküle in der Reaktion | Verdünnung 1:4000 | Signal/Rauschen 1:40 | 1:10 |
|---|---|---|---|
| 1 | 1 | 2 | 10 |
| 72 | 1 | 7 | 30 |
| 7,2x10³ | 5 | 150 | |
| 7,2x10⁵ | 40 | 1013 | |
| 7,2x10⁷ | 71 | | |
| 7,2x10⁹ | 111 | | |

### Beispiel 17: Amplifizierung eines Abschnittes aus einem ungeschnittenen Plasmid mit Primern, wobei das Amplifizierungsprodukt länger ist als die Summe der Basen der beiden Oligonukleotidbausteine:

Als Template-DNA dient das Plasmid pUC19, das nicht linearisiert wurde. Als Oligonukleotidbausteine wurden pUC19:20U1897 (5'-dAgTAgTTCgCCAgTTAATAg-3') und pUC19:18L1926 (5'-dgTAgCAATggCAACAACg-3') verwendet.

Es wurden 50 µl Reaktionsansätze in 0,5 ml Reaktionsgefäßen (Eppendorf, Safe-Lock) zusammenpipettiert, wobei die Reaktionsansätze die folgende Zusammensetzung aufwiesen.
20 mM Tris-HCl (pH 8,8 bei 25 ° C)
5 mM MgSO₄
je 200 µM TTP, dGTP, dATP, dCTP
0,1% (M/V) Triton X-100
10 pmol Oligonukleotidbaustein pUC19:20U1897
10 pmol Oligonukleotidbaustein pUC19:18L1926
1 ng Template-DNA

Als Negativkontrolle wurde der gleiche Ansatz wie oben pipettiert, mit dem Unterschied, daß die Template DNA weggelassen wurde. Anschließend wurden die Reaktionsansätze mit 100 µl Mineralöl (SIGMA, M-3516) überschichtet. Die Ansätze wurden in einem Thermostatisiergerät (Gene Amp PCR System 9600, Perkin Elmer Cetus) auf 74° C erwärmt, anschließend jeweils 2 Units des Enzyms Deep Vent_{R}(exo⁻) DNA-Polymerase (New England Biolabs) zugesetzt und 1 Stunde inkubiert.

10 µl der Reaktionsgemische wurden auf einem 2%igen Agarosegel aufgetrennt und durch Färbung mit Ethidiumbromid und UV-Fluoreszenzanalyse sichtbar gemacht. Das Amplifizierungsprodukt konnte durch Vergleich mit einem DNA-Längenstandard als einzige Bande identifiziert werden.

## Patentansprüche

1. Verfahren zur transkriptionsfreien exponentiellen Amplifizierung von Nukleinsäuren bzw. Nukleinsäuresequenzen mittels Enzymen, wobei die Nukleinsäuren bzw. Nukleinsäuresequenzen gegebenenfalls vor der Amplifizierung zumindest teilweise in ihre Einzelstränge aufgetrennt und/oder transkribiert werden, und in der Reaktionsmischung Oligonukleotide mit einer Basensequenz im wesentlichen komplementär zu den Basensequenzen der Enden der zu amplifizierenden Nukleinsäure bzw. Nukleinsäuresequenz enthalten sind, welche Oligonukleotide unter geeigneten Bedingungen Startpunkte und/oder chemische Bausteine zur Nukleinsäuresynthese bilden können und in das zu bildende Amplifizierungsprodukt eingebaut werden, wobei das Produkt aus den Bausteinen selbst wieder der zu amplifizierenden Nukleinsäure bzw. Nukleinsäuresequenz entspricht, dadurch gekennzeichnet, daß
- das Reaktionsprodukt, welches aus den Oligonukleotidbausteinen, gegebenenfalls dem Template und gegebenenfalls aus weiteren chemischen Bausteinen gebildet wurde, in einem Schritt wieder mit den Oligonukleotidbausteinen reagiert,
- daß die Reaktion bei einer die Doppelstrangform des Reaktionsproduktes destabilisierenden Temperatur im wesentlichen isotherm geführt wird und
- daß bei Verwendung von Polymerase als Enzym diese Strangverdrängungsaktivität aufweist.

2. Verfahren nach Anspruch 1, dadurch gekennzeichnet, daß das bei der Amplifizierung verwendete Enzym eine Polymerase ist.

3. Verfahren nach Anspruch 2, dadurch gekennzeichnet, daß die bei der Amplifizierung verwendete Polymerase auch Reverse Transkriptase-Aktivität hat.

4. Verfahren nach Anspruch 2 oder 3, dadurch gekennzeichnet, daß bei der Amplifizierung als Enzym außer Polymerase zusätzlich Ligase verwendet wird.

5. Verfahren nach Anspruch 1, dadurch gekennzeichnet, daß das bei der Amplifizierung verwendete Enzym eine Ligase ist.

6. Verfahren nach einem der Ansprüche 1 bis 5, dadurch gekennzeichnet, daß das verwendete Oligonukleotid an einem geeigneten Träger, wie einer Kunststoffoberfläche, immobilisiert ist.

7. Verfahren nach einem der Ansprüche 1 bis 6, dadurch gekennzeichnet, daß die Amplifizierung bei einer Temperatur oberhalb der Schmelztemperatur der verwendeten Oligonukleotide durchgeführt wird.

8. Verfahren nach einem der Ansprüche 1 bis 7, dadurch gekennzeichnet, daß die Oligonukleotide am Template möglichst nahe beisammen liegen.

9. Verfahren nach einem der Ansprüche 1 bis 8, dadurch gekennzeichnet, daß Hilfsoligonukleotide zur Verbesserung der Amplifizierung eingesetzt werden.

10. Verfahren nach einem der Ansprüche 1 bis 9, dadurch gekennzeichnet, daß die Oligonukleotidsequenz zumindest zu einem Teil der Templatesequenz exakt komplementär ist.

11. Verwendung des Verfahrens nach einem der Ansprüche 1 bis 10 für diagnostische Methoden außerhalb des menschlichen oder tierischen Körpers.

12. Verwendung eines Verfahrens nach einem der Ansprüche 1 bis 10 als Amplifizierungsschritt in anderen Nukleinsäureamplifizierungsverfahren.

## Claims

1. A process for the transcription-free exponential amplification of nucleic acids or nucleic acid sequences, respectively, by means of enzymes, wherein the nucleic acids or nucleic acid sequences, respectively, are optionally at least partially separated into their single strands and/or transcribed prior to amplification, and oligonucleotides having a base sequence essentially complementary to the base sequences of the ends of the nucleic acid or nucleic acid sequence, respectively, to be amplified are contained in the reaction mixture, which oligonucleotides may, under suitable conditions, constitute starting points and/or chemical building blocks for nucleic acid synthesis and are incorporated into the amplification product to be formed, wherein the product itself to be formed from the building blocks in turn corresponds to the nucleic acid to be amplified or to the nucleic acid sequence, respectively,
characterized in that
- the reaction product which has been formed from the oligonucleotide building blocks, optionally the template and optionally further chemical building blocks, in a further step again reacts with the oligonucleotide building blocks,
- that the reaction is carried out substantially isothermally at a temperature destabilizing the double-stranded form of the reaction product, and
- that if polymerase is used as enzyme, the latter comprises a strand displacement activity.

2. A process according to claim 1, characterized in that the enzyme used in the amplification is a polymerase.

3. A process according to claim 2, characterized in that the polymerase used in the amplification also comprises reverse transcriptase activity.

4. A process according to claim 2 or 3, characterized in that at the amplification, besides polymerase, ligase is additionally used as enzyme.

5. A process according to claim 1, characterized in that the enzyme used at the amplification is a ligase.

6. A process according to any one of claims 1 to 5, characterized in that the oligonucleotide used is immobilized on a suitable carrier, such as a synthetic material surface.

7. A process according to any one of claims 1 to 6, characterized in that amplification is carried out at a temperature above the melting temperature of the oligonucleotides used.

8. A process according to any one of claims 1 to 7, characterized in that the oligonucleotides are located on the template as closely to each other as possible.

9. A process according to any one of claims 1 to 8, characterized in that auxiliary oligonucleotides are used to improve amplification.

10. A process according to any one of claims 1 to 9, characterized in that the oligonucleotide sequence at least in part is exactly complementary to the template sequence.

11. The use of the process according to any one of claims 1 to 10 for diagnostic methods externally of the human or animal body.

12. The use of a process according to any one of claims 1 to 10 as amplification step in other nucleic acid amplification processes.

## Revendications

1. Procédé d'amplification exponentielle sans transcription d'acides nucléiques ou de séquences d'acides nucléiques à l'aide d'enzymes, dans lequel les acides nucléiques ou les séquences d'acides nucléiques sont éventuellement séparés au moins partiellement en leurs brins individuels et/ou transcrits avant l'amplification, et le mélange réactionnel contient des oligonucléotides ayant une séquence de bases essentiellement complémentaire des séquences de bases des extrémités de l'acide nucléique ou de la séquence d'acide nucléique à amplifier, lesquels oligonucléotides peuvent former dans des conditions appropriées des points de départ et/ou des éléments constitutifs chimiques pour la synthèse d'acides nucléiques et s'incorporent dans le produit d'amplification à former, le produit obtenu à partir des éléments constitutifs correspondant lui-même de nouveau à l'acide nucléique ou à la séquence d'acide nucléique à amplifier, caractérisé en ce que
- le produit réactionnel formé à partir des éléments constitutifs oligonucléotidiques, éventuellement de la matrice et éventuellement d'autres éléments constitutifs chimiques, réagit de nouveau en une étape avec les éléments cosntitutifs oligonucléotidiques,
- l'on effectue la réaction de façon essentiellement isotherme à une température qui déstabilise la forme double brin du produit réactionnel, et,
- lorsque l'on utilise une polymérase comme enzyme, celle-ci présente une activité de déplacement des brins.

2. Procédé selon la revendication 1, caractérisé en ce que l'enzyme utilisée dans l'amplification est une polymérase.

3. Procédé selon la revendication 2, caractérisé en ce que la polymérase utilisée dans l'amplification a aussi une activité de transcriptase inverse.

4. Procédé selon la revendication 2 ou 3, caractérisé en ce que, lors de l'amplification, outre la polymérase, on utilise en plus une ligase comme enzyme.

5. Procédé selon la revendication 1, caractérisé en ce que l'enzyme utilisée dans l'amplification est une ligase.

6. Procédé selon l'une des revendications 1 à 5, caractérisé en ce que l'oligonucléotide utilisé est immobilisé sur un support approprié comme une surface de matière plastique.

7. Procédé selon l'une des revendications 1 à 6, caractérisé en ce que l'amplification s'effectue à une température supérieure à la température de fusion des oligonucléotides utilisés.

8. Procédé selon l'une des revendications 1 à 7, caractérisé en ce que les oligonucléotides sont ensemble le plus proches possible de la matrice.

9. Procédé selon l'une des revendications 1 à 8, caractérisé en ce que l'on utilise des oligonucléotides auxiliaires pour améliorer l'amplification.

10. Procédé selon l'une des revendications 1 à 9, caractérisé en ce que la séquence des oligonucléotides est exactement complémentaire d'au moins une partie de la séquence de la matrice.

11. Utilisation du procédé selon l'une des revendications 1 à 10 pour des méthodes de diagnostic s'effectuant en dehors du corps humain ou animal.

12. Utilisation d'un procédé selon l'une des revendications 1 à 10 comme étape d'amplification dans d'autres procédés d'amplification d'acides nucléiques.
